(19) Europäisches Patentamt
European Patent Office
Office européen des brevets

(11) Veröffentlichungsnummer : **0 355 492 B1**

(12) # EUROPÄISCHE PATENTSCHRIFT

(45) Veröffentlichungstag der Patentschrift :
**09.12.92 Patentblatt 92/50**

(51) Int. Cl.$^5$ : **C09B 62/503,** C07C 317/18, D06P 1/384

(21) Anmeldenummer : **89114165.7**

(22) Anmeldetag : **01.08.89**

(54) **Reaktivfarbstoffe.**

(30) Priorität : **13.08.88 DE 3827530**

(43) Veröffentlichungstag der Anmeldung :
**28.02.90 Patentblatt 90/09**

(45) Bekanntmachung des Hinweises auf die Patenterteilung :
**09.12.92 Patentblatt 92/50**

(84) Benannte Vertragsstaaten :
**CH DE FR GB LI**

(56) Entgegenhaltungen :
**EP-A- 0 158 857**
**EP-A- 0 271 656**

(73) Patentinhaber : **BAYER AG**
**W-5090 Leverkusen 1 Bayerwerk (DE)**

(72) Erfinder : **Harms, Wolfgang, Dr.**
**Osenauer Strasse 46**
**W-5068 Odenthal (DE)**

**EP 0 355 492 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus der EP-A-0 271 656 sind bereits Amino-alkoxy-triphendioxazin-Reaktivfarbstoffe bekannt, die als reaktive Gruppen vorzugsweise Triazine aufweisen.

Die vorliegende Erfindung betrifft neue Triphendioxazin-Reaktivfarbstoffe der allgemeinen Formel

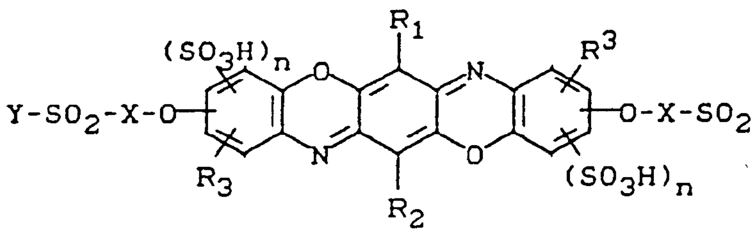

(I),

worin

X aliphatisches Brückenglied

Y $-CH=CH_2$, $-CH_2-CH_2-Z$,

worin

Z abspaltbare Gruppe wie $OSO_3H$, $S_2O_3H$, Cl, Br, $O-COCH_3$, $OPO_3H_2$, $\overset{\ominus}{N}(R_4)_3$ mit $R_4$ = $C_1$-$C_4$-Alkyl, bevorzugt jedoch $OSO_3H$ ist,

n 0 - 2, vorzugsweise 1,

$R_1$, $R_2$ H, Halogen, $C_1$-$C_4$-Alkoxy, gegebenenfalls substituiertes Phenoxy oder Aryl, Acylamino, Carboxy, Carbalkoxy, vorzugsweise Cl,

$R_3$ H, $C_1$-$C_4$-Alkyl, $C_1$-$C_4$-Alkoxy, Halogen, Carboxy

bedeuten.

Beispiele für aliphatische Brückenglieder X sind unverzweigte oder verzweigte, gegebenenfalls substituierte oder durch Heteroatome bzw. O oder N oder solche enthaltende Gruppen unterbrochene $C_2$-$C_8$-Alkylenreste wie Ethylen, 1,3- oder 1,2-Propylen, 1,4-, 1,3-, 2,3-Butylen, 1,5-Pentylen, 1,6-Hexylen, 2,2-Dimethyl-1,3-propylen, 2-Ethyl-1,3-propylen, 1,4-, 1,3-Cyclohexylen, 2-Hydroxy-1,3-propylen, 2-Oxo-1,3-propylen, 2-Sulfato-1,3-propylen, $-CH_2-CH_2-O-CH_2-CH_2-$, $-(CH_2-CH_2-O)_{2\text{-}3}-CH_2-CH_2-$,

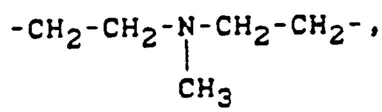

$-CH_2-CH_2-NH-CH_2-CH_2-$,

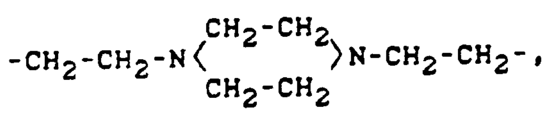

$-CH_2-CH_2-CO-NH-CH_2-CH_2-$, $-CH_2-CO-NH-CH_2-CH_2-$, $-CH_2-CH_2-NH-CO-CH_2-$, $-CH_2-CH_2-NH-CO-CH_2-CH_2-$.

Bevorzugte Farbstoffe der Formel (I) sind solche der Formel

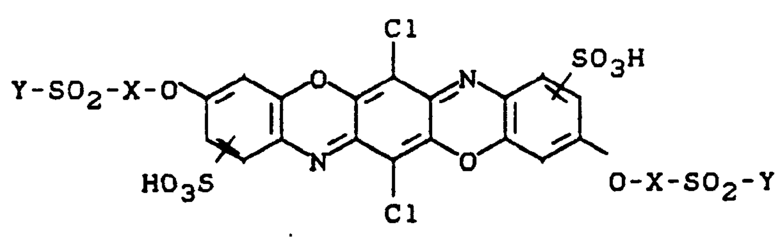

(II),

insbesondere solche der Formel

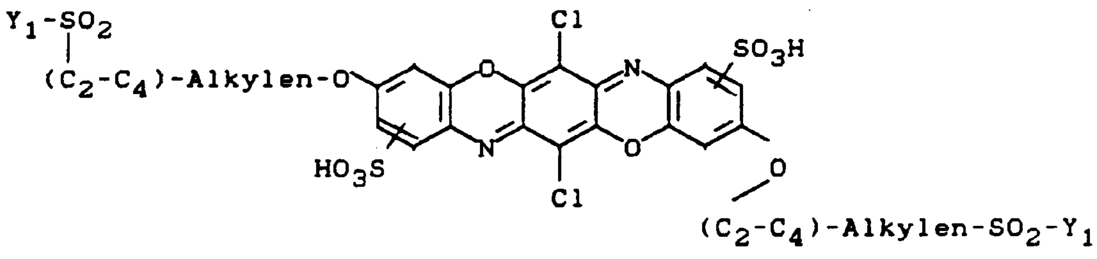

(III),

worin
$Y_1$ $CH_2$-$CH_2$-$OSO_3H$ oder -CH=$CH_2$
bedeutet.

Ganz generell befinden sich im Triphendioxazinringsystem vorhandene Sulfogruppen, vorzugsweise in o-Stellung zum Rest -O-X-$SO_2$-Y.

Die Farbstoffe der Formel (I) werden hergestellt durch Kondensation von ω-(Aminophenoxy)-alkyl-β'-hydroxy- oder -β'-sulfatoethylsulfonen der Formeln

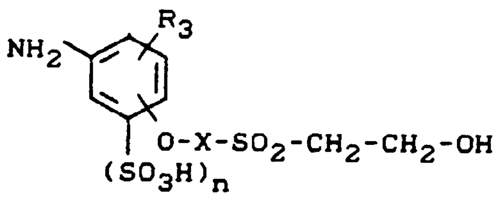

(IV)

oder

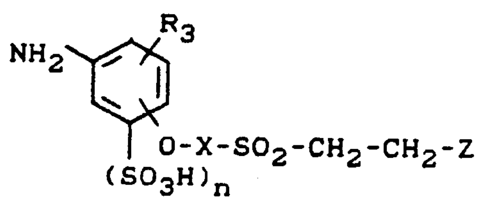

(V)

worin
Z, X, $R_3$ und n die oben angegebene Bedeutung haben und
n 0, 1 oder 2 bedeutet,
mit 1,4-Benzochinonen der Formel

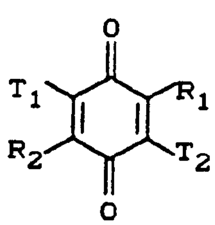

(VI)

worin
$T_1$, $T_2$ Wasserstoff, Cl, Br, O-($C_1$-$C_4$)-Alkyl, gegebenenfalls substituiertes O-Phenyl (Substituenten insbesondere Cl, Nitro, $C_1$-$C_4$-alkyl) bedeuten zu Verbindungen der Formel

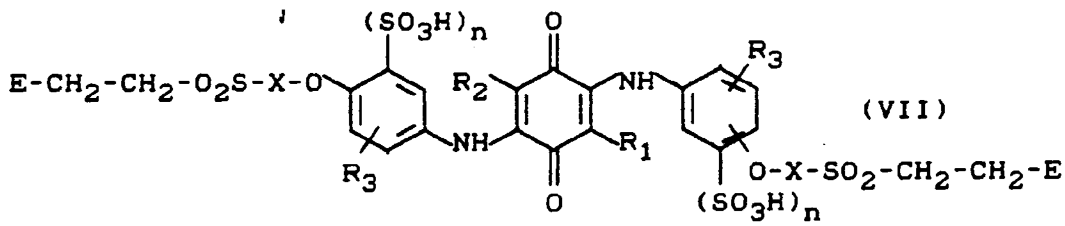

(VII)

worin
E = OH oder Z und
Z, $R_1$, $R_2$, $R_3$, X und n die oben angegebene Bedeutung haben
und nachfolgenden Ringschluß der Dianilide (VII) zu den Triphendioxazin-Verbindungen der Formel (I). Im Zuge dieser letzten Operation können in üblicher Weise Sulfatogruppen und gegebenenfalls Sulfonsäuregruppen oder weitere Sulfonsäuregruppen eingeführt werden. Gegebenenfalls können anschließend auch funktionelle Umwandlungen der $-CH_2-CH_2-OSO_3H$-Grupen zu beispielsweise $-CH=CH_2$- oder $-CH_2-CH_2-S_2O_3H$-Gruppen vorgenommen werden.

Je nach den beim Ringschluß angewandten Reaktionsbedingungen können die Sulfonsäuregruppen in den Benzringen der Dioxazine (I) entweder in den o-oder in den p-Stellungen zu den Ringsauerstoffatomen des Dioxazin-Systems auftreten.

Die Kondensation der Benzochinone der Formel (VI) mit den Aminen (IV) oder (V) erfolgt vorzugsweise in wäßrigem oder wäßrig-organischem Medium unter Zugabe alkalischer Kondensationsmittel bei pH-Werten von 3-11, vorzugsweise 4-8, und Temperaturen von 20-90°C, vorzugsweise 40-70°Coder in gepufferten Lösungen, die obige alkalische Kondensationsmittel enthalten. Man kann auch in rein organischem Milieu unter Zusatz säurebindender Mittel arbeiten.

Alkalische Kondensationsmittel sind beispielsweise Natriumhydrogencarbonat, Natriumcarbonat, Natrium- oder Kaliumacetat, Natronlauge, Kalilauge, Natriumphosphate, Natriumborat.

Im allgemeinen fallen die Kondensationsprodukte der Formel (VII) als schwerlösliche, hellbraune Produkte aus.

Eine Variante zur Herstellung von Verbindungen der Formel (VII) besteht in der Addition von Aminen (IV) oder (V) an 1,4-Benzochinone der Formel

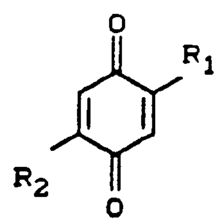

(VIII)

worin
$R_1$ und $R_2$ die oben angegebene Bedeutung haben,
und Oxydation der primär entstehenden Addukte.

Der Ringschluß der Chinonkondensationsprodukte (VII) zu den Dioxazinen (I) kann nach an sich bekannten Methoden, wie sie in den Deutschen Offenlegungsschriften 2 122 262, 2 124 080, 2 302 383, 2 344 781, 2 503 611, 2 823 828 und in der Britischen Patentschrift 2 019 872 beschrieben sind, insbesondere in konzentrierter Schwefelsäure und vor allem in Oleum mit $SO_3$-Gehalten von 1-50 % bei Temperaturen von 10-80°C gegebenenfalls mit Zusatz von Oxidationsmitteln wie Kalium- oder Ammoniumperoxidisulfat, Braunstein oder organischen Peroxiden oder mit Jod oder jodabgebenden Mitteln wie Kaliumjodid vorgenommen werden.

Amine der Formel (IV) bzw. (V) sind neu und ebenfalls Gegenstand der Erfindung. Sie lassen sich beispielsweise folgendermaßen herstellen:

Nitroverbindungen oder Acylaminoverbindungen der Formel

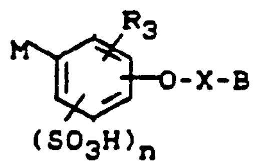

(IX)

worin

M $NO_2$, Acylamino, insbesondere $C_1$-$C_4$-Alkylcarbonylamino, gegebenenfalls substituiertes Phenylcarbonylamino, Carboxy-$C_2$-$C_4$-carbonylamino,

B Halogen, insbesondere Cl, Br, -$OSO_2$-Aryl (vorzugsweise Phenyl und $C_1$-$C_4$-Alkylphenyl), -$OSO_2$-$C_1$-$C_4$-alkyl bedeuten,

werden in an sich bekannter Weise mit 2-Mercaptoethanol zu Sulfiden der Formel

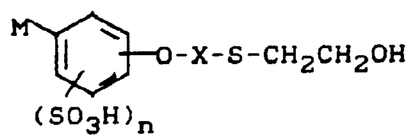

(X)

umgesetzt und diese in bekannter Weise, beispielsweise mit $H_2O_2$ oder Chlor zu Sulfonen der Formel

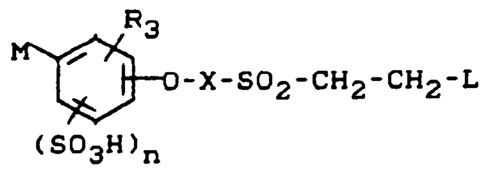

(XI)

worin

L = OH, Cl,

oxydiert und anschließend die Nitrogruppe M zur Aminogruppe reduziert bzw. die Acylaminogruppe M verseift und gegebenenfalls anschließend die OH-Gruppe L in eine Gruppe -$OSO_3H$, -$S_2O_3H$, -$OPO_3H_2$ umwandelt und/oder in den Benzolring Sulfogruppen einführt (in bekannter Weise mit Schwefelsäure, Oleum, Schwefeltrioxid oder Chlorsulfonsäure).

Eine weitere spezielle Methode zur Herstellung von Verbindungen der Formel (X) mit X = -$CH_2$-$CH_2$- besteht in der halbseitigen Umsetzung von 2,2'-Bis-(hyroxyethyl)-sulfid mit Nitrohalogenverbindungen der Formel

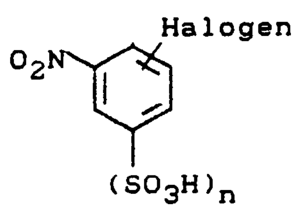

(XII)

worin

Halogen F, Cl, Br bedeutet

und

n die oben angegebene Bedeutung hat.

Gegenstand der vorliegenden Erfindung sind auch die neuen Verbindungen der Formel

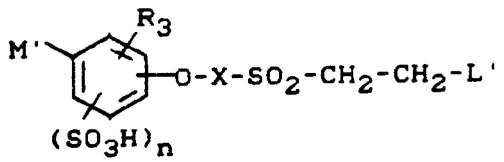

(XIII)

worin

M' Nitro, Acylamino, $NH_2$

L' OH oder anionisch abspaltbare Gruppe beispielsweise $-OSO_3H$, $-S_2O_3H$, $-OPO_3H_2$, Cl

$R_3$ H, $C_1$-$C_4$-Alkyl, $C_1$-$C_4$-Alkoxy, Halogen (Cl, Br), Carboxy

X aliphatisches Brückenglied

n 0, 1 oder 2 bedeuten.

Für M' $NO_2$ ist dabei n vorzugsweise 0.

Geeignete Aminoverbindungen der Formeln (IV) und (V) zur Umsetzung mit den Chinonen (VI) oder (VIII) sind beispielsweise:

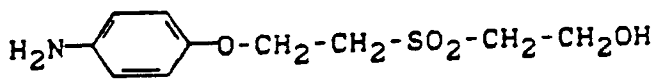

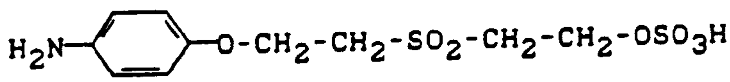

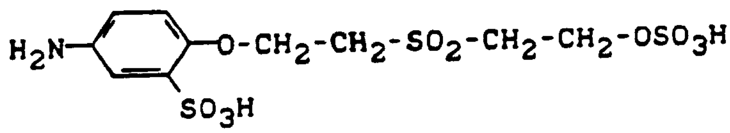

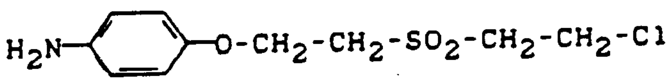

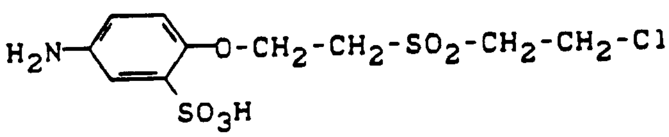

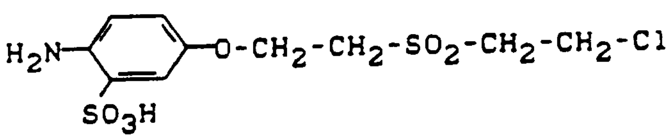

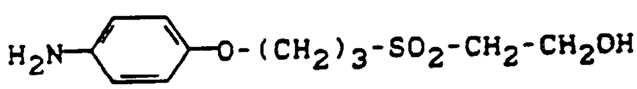

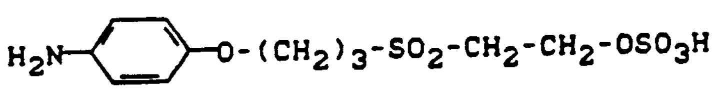

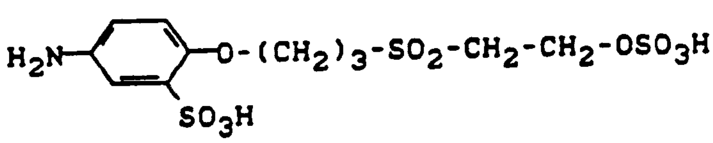

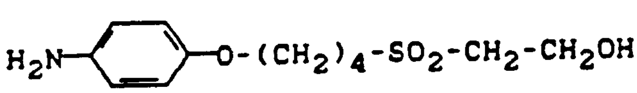

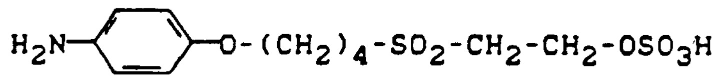

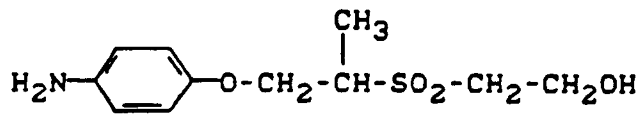

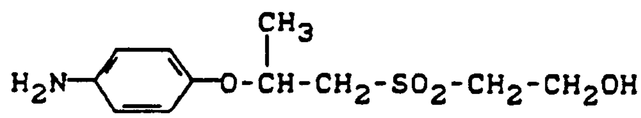

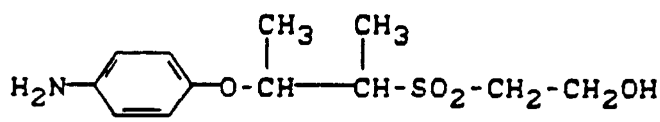

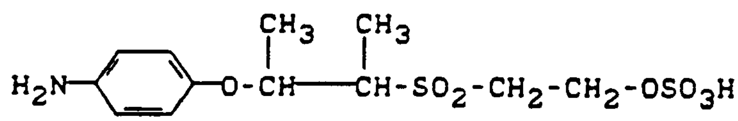

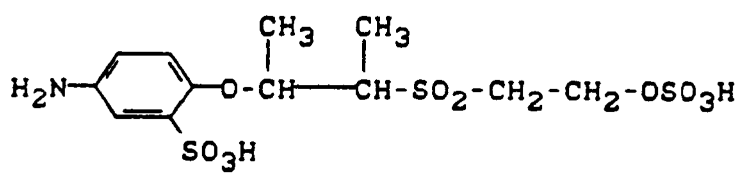

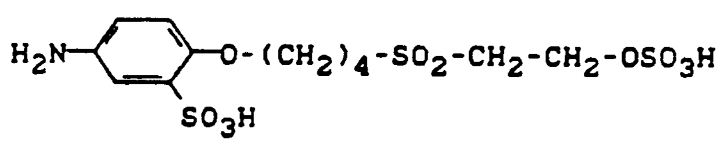

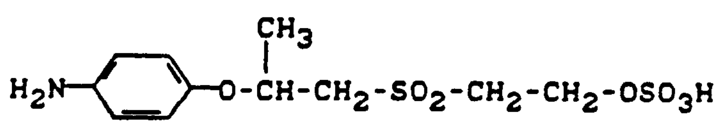

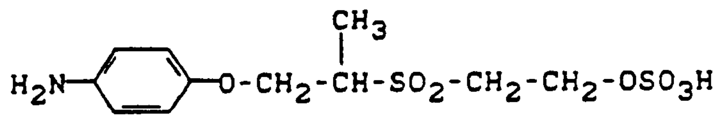

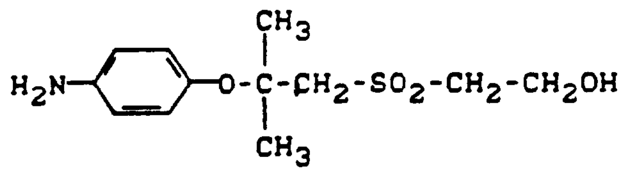

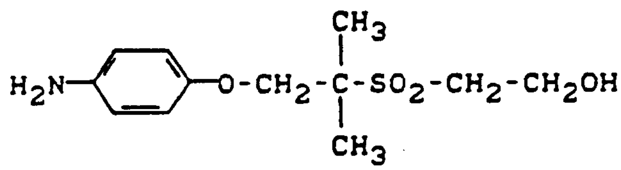

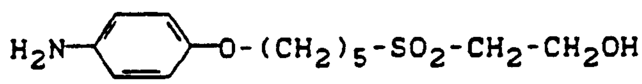

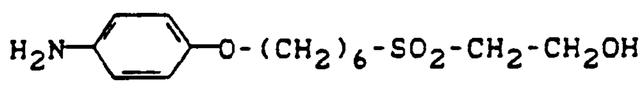

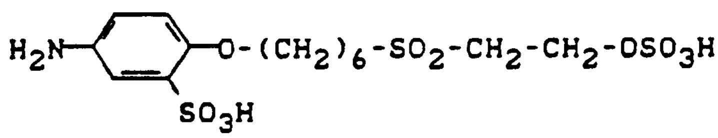

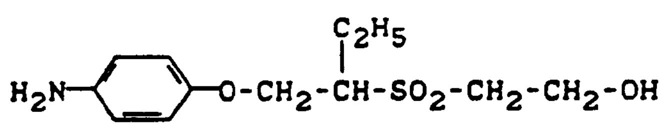

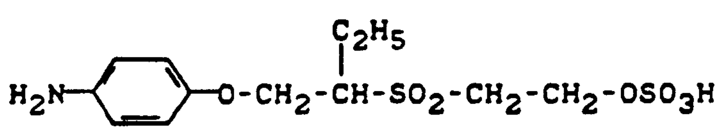

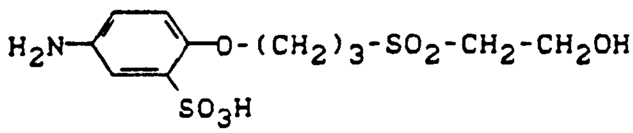

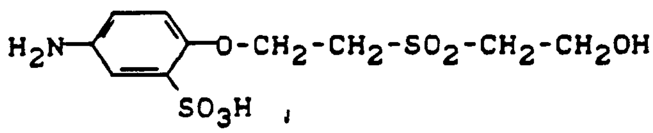

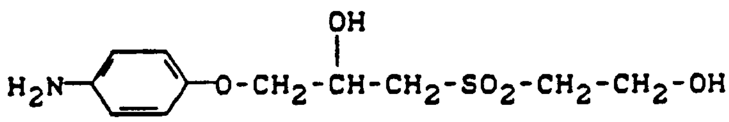

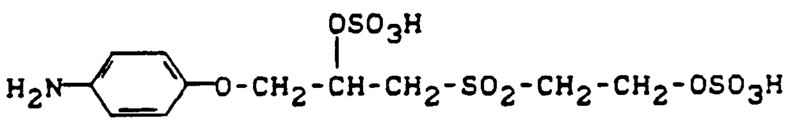

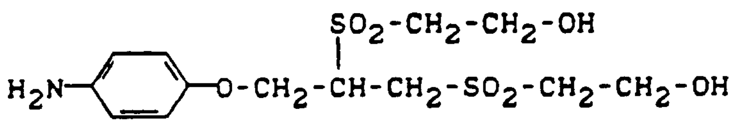

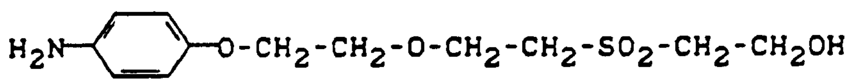

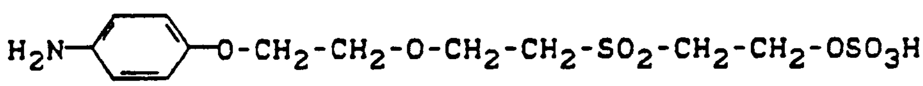

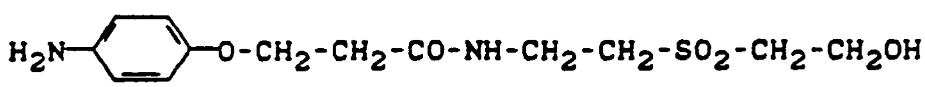

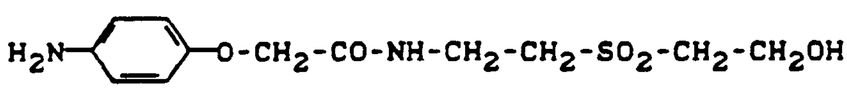

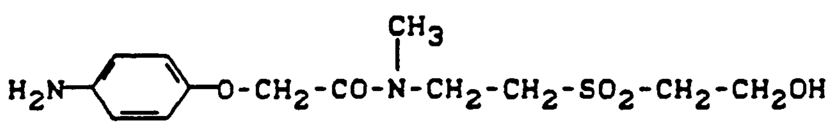

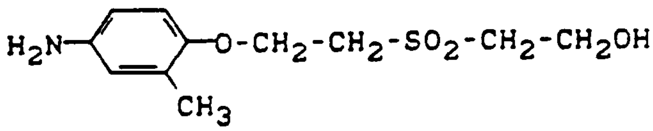

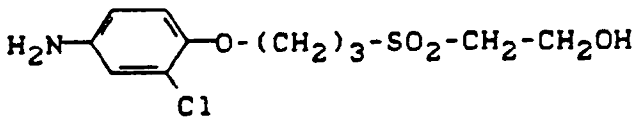

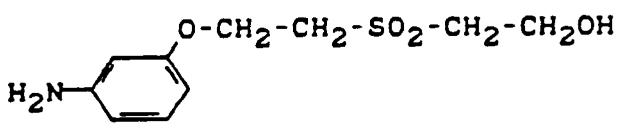

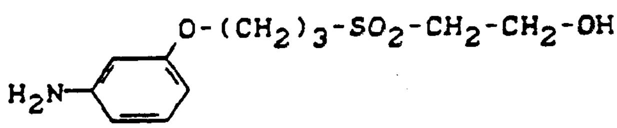

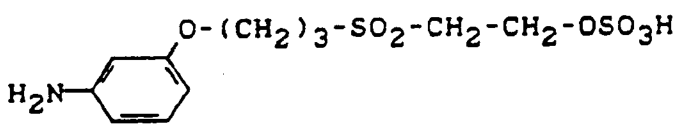

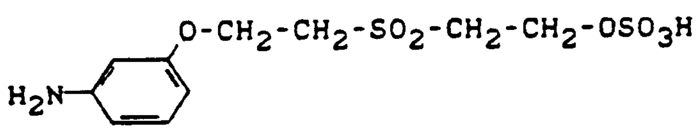

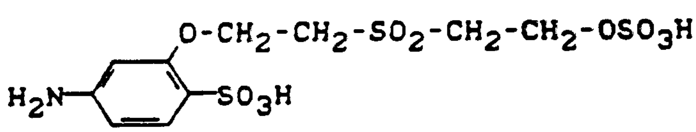

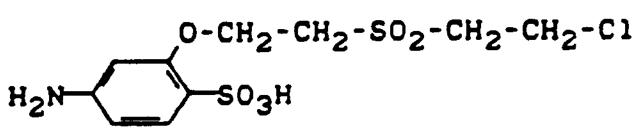

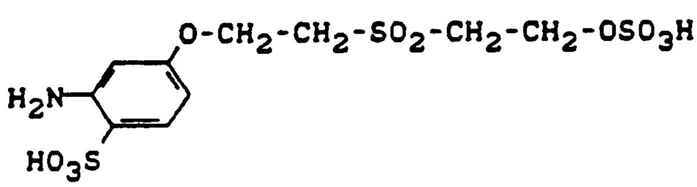

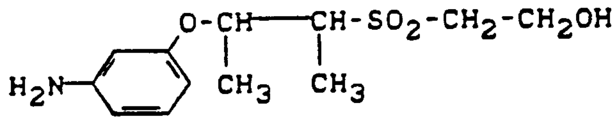

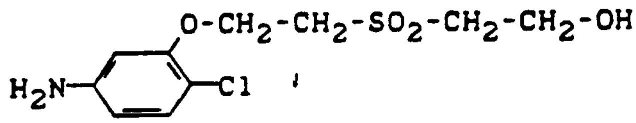

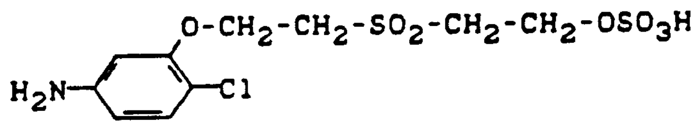

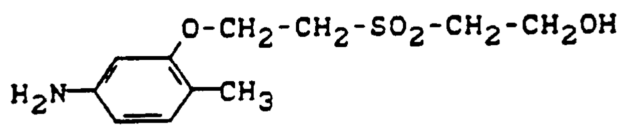

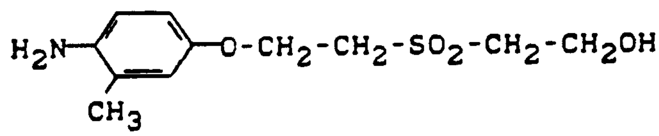

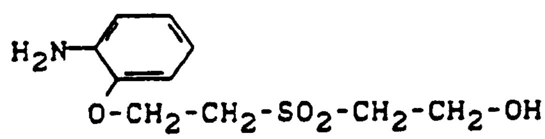

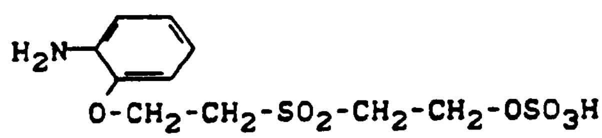

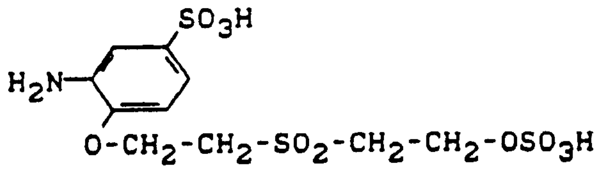

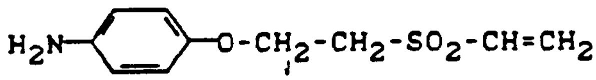

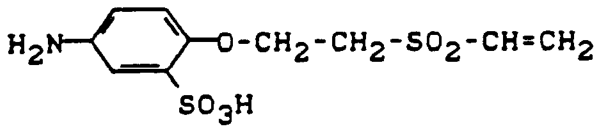

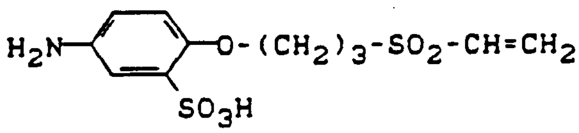

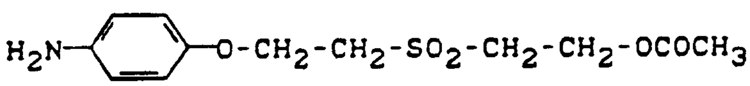

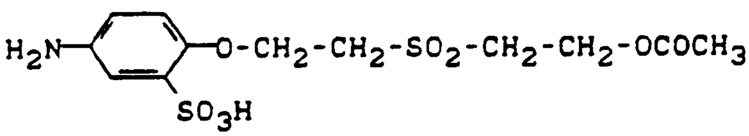

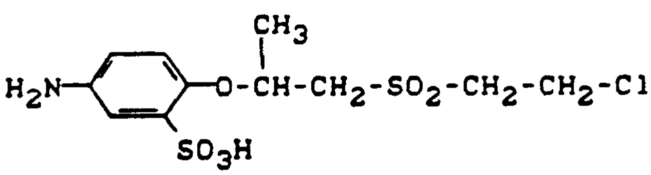

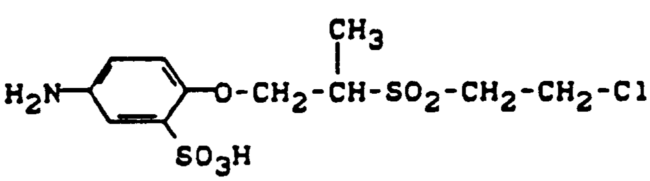

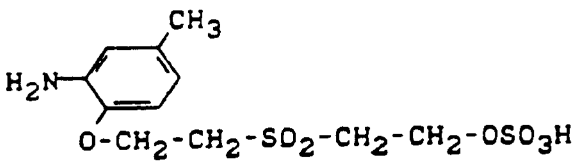

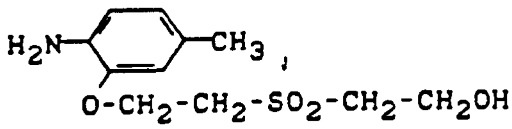

Für die Kondensation mit den Aminen der Formel (IV) oder (V) geeignete 1,4-Benzochinone (VI) oder (VIII) sind beispielsweise

2,3,5,6-Tetrachlor-1,4-benzochinon
2,3,5,6-Tetrabrom-1,4-benzochinon
2,5-Dichlor-3,6-dimethyl-1,4-benzochinon
2,5-Dichlor-3,6-dimethoxy-1,4-benzochinon
2,3,5,6-Tetramethoxy-1,4-benzochinon,
2,3,5,6-Tetraphenoxy-1,4-benzochinon,
2,3,5,6-Tetra-(4′-methylphenoxy)-1,4-benzochinon,
2,3,5,6-Tetra-(4′-methoxyphenoxy)-1,4-benzochinon,
2,5-Diacetylamino-3,6-dichlor-1,4-benzochinon,
2,5-Dibenzoylamino-3,6-dichlor-1,4-benzochinon,
2,3,5,6-Tetra-(4′-chlorphenoxy)-1,4-benzochinon,
2,3,5,6-Tetra-4-(3′-methyl-4′-chlorphenoxy)-1,4-benzochinon,
2-Ethyl-3,6-dimethoxy-1,4-benzochinon,
2-Chlor-3,6-dimethoxy-1,4-benzochinon,
2,3,5-Trimethoxy-1,4-benzochinon,
2,3,5,6-Tetra-(4′-nitrophenoxy)-1,4-benzochinon,
2,5-Dimethyl-3,6-dimethoxy-1,4-benzochinon,
2-Methyl-3,6-dimethoxy-1,4-benzochinon,
2-Methyl-5,6-dimethoxy-1,4-benzochinon,
2-Ethyl-3,6-dimethoxy-1,4-benzochinon,
2-Chlor-3-n-propyl-5-methoxy-1,4-benzochinon,
2-Chlor-3,5-dimethoxy-1,4-benzochinon,
2-Methyl-3,5-dichlor-1,4-benzochinon,
2-Methyl-3,5,6-tribrom-1,4-benzochinon,
2-(4′-Methylphenoxy)-3,6-dibrom-1,4-benzochinon,
2-(3′-Methylphenoxy)-3,6-dibrom-1,4-benzochinon,
2-Methyl-3,5,6-trichlor-1,4-benzochinon,
2-Methyl-3-chlor-5-brom-1,4-benzochinon,
2-Methyl-3,6-dichlor-1,4-benzochinon,
2-Methyl-3,6-dichlor-5-brom-1,4-benzochinon,
2-Phenyl-3,6-dichlor-1,4-benzochinon,
2-(4′-Methoxyphenyl)-3,6-dichlor-1,4-benzochinon,
2-(4′-Chlorphenyl)-3,6-dichlor-1,4-benzochinon,
2-(4′-Nitrophenyl)-3,6-dichlor-1,4-benzochinon,
2-(4′-Nitrophenyl)-3,5,6-trichlor-1,4-benzochinon,
2,5-Dimethyl-3,6-dibrom-1,4-benzochinon,
2,5-Dimethyl-3-chlor-1,4-benzochinon,
2-Methyl-5-n-propyl-6-brom-1,4-benzochinon,
2-Methyl-5-isopropyl-3-chlor-1,4-benzochinon,
2-Methyl-5-isopropyl-6-brom-1,4-benzochinon und
2-(2′-Chlorphenyl)-3,5,6-tribrom-1,4-benzochinon,
2-Methyl-3-methoxy-1,4-benzochinon,
1,4-Benzochinon,
2-Methyl-1,4-benzochinon,
2-Ethyl-1,4-benzochinon,
2-n-Propyl-1,4-benzochinon,
2-Isopropyl-1,4-benzochinon,
2,2′-Ethoxyethyl-1,4-benzochinon,
2-Phenyl-1,4-benzochinon,
2-(4′-Methylphenyl)-1,4-benzochinon,

2-(4'-Methoxyphenyl)-1,4-benzochinon,
2-(3'-Chlorophenyl)-1,4-benzochinon,
2-(4'-Nitrophenyl)-1,4-benzochinon,
2,5-Dimethyl-1,4-benzochinon,
2-Methyl-5-ethyl-1,4-benzochinon,
2-Methyl-3-chlor-1,4-benzochinon,
2-Methyl-6-chlor-1,4-benzochinon,
2,5-Dichlor-3,6-di-(methoxcarbonyl)-1,4-benzochinon,
2,5-Dibrom-3,6-di-(ethoxycarbonyl)-1,4-benzochinon,
2,3,5,6-Tetrafluor-1,4-benzochinon.

Umsetzungen derartiger 1,4-Benzochinone des Typs (VI) oder (VIII) sind ausführlich in der Deutschen Offenlegungsschrift 2 823 828 beschrieben.

Die neuen Farbstoffe sind wertvolle Produkte, die sich durch hohe Farbstärken auszeichnen. Sie eignen sich in dispergierter oder gelöster Form für die verschiedensten Anwendungszwecke.

Als wasserlösliche Verbindungen finden sie bevorzugtes Interesse für das Färben hydroxyl- und amidgruppenhaltiger Textilmaterialien, insbesondere von Materialien aus nativer und regenerierter Cellulose sowie synthetischen Polyamid- und Polyurethanfasern, Wolle und Seide.

Als wasserlösliche Reaktivfarbstoffe werden die genannten Materialien nach den für Reaktivfarbstoffe allgemein üblichen Verfahren gefärbt oder bedruckt. Man erhält dann licht- und naßechte rote Färbungen und Drucke.

Bei Temperaturangaben in den Beispielen handelt es sich um °C. Die Formeln der wasserlöslichen Farbstoffe in der Beschreibung und in den Beispielen sind die der freien Säuren. Die Farbstoffe werden in der Regel in Form ihrer Alkalisalze, insbesondere der Lithium-, Natrium- oder Kaliumsalze, isoliert und angewandt.

Die neuen Verbindungen (IV) und (V) bzw. (XIII) sind wertvolle Zwischenprodukte für die Herstellung von Farbstoffen. Die Verbindungen (IV) und (V) sind Zwischenprodukte für die Herstellung von Farbstoffen (I). Die Verbindungen (XIII) mit M' = $NH_2$ sind beispielsweise als Diazokomponenten zur Herstellung von Azofarbstoffen geeignet.

Beispiel 1

42,8 g 3-(4-Aminophenoxy)-propyl-2-hydroxyethylsulfon werden in 430 ml Wasser und 65 ml Isopropanol suspendiert. Man erwärmt auf 40°, stellt den pH-Wert auf 6,0 ein und fügt 20,3 g 2,3,5,6-Tetrachlorbenzochinon hinzu. Man hält bei einer Temperatur von 40° den pH-Wert im Reaktionsgemisch mit 2n Sodalösung beständig auf 5,8-6,0 und hält diese Bedingungen mehrere Stunden aufrecht, bis die Sodaaufnahme zum Stillstand kommt.

Der feinkristalline hellbraune Niederschlag wird abgesaugt, mit 500 ml Wasser und 250 ml Methanol gewaschen und bei 60° im Umluftschrank getrocknet. Das erhaltene Produkt entspricht der Formel

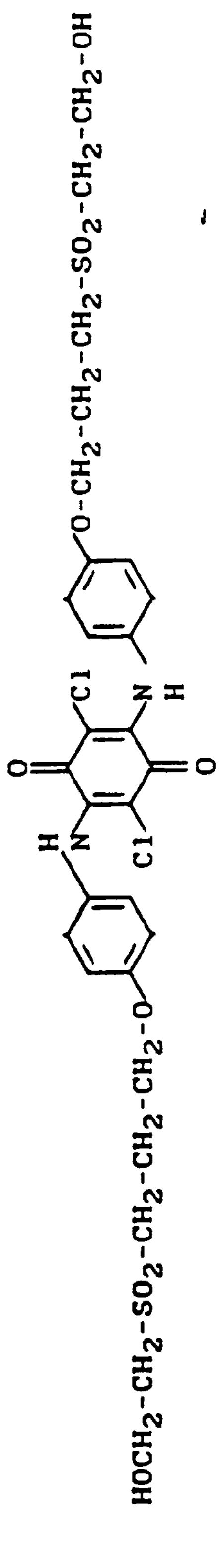

30,0 g des erhaltenen Zwischenproduktes werden in 100 ml 26 %iges Oleum bei -5 bis 0° in 2-3 Stunden gleichmäßig eingetragen. Dann erhöht man die Temperatur auf +20°C und rührt einige zeit nach, bis eine chromatographische Probe keine Veränderung mehr anzeigt und in das Molekül neben 2 Sulfatogruppen außerdem 2 Sulfonsäuregruppen eingetreten sind. Man erwärmt den Ansatz auf 30° und trägt im Laufe einer Stunde 23,5 g Kaliumperoxodisulfat ein, hält dabei die Temperatur zwischen 28 und 33° und rührt noch 30 Minuten bis zur vollständigen Reaktion bei 30° weiter.

Die entstandene blaue Lösung wird in 600 g Eis und 100 ml Wasser eingetragen, die braune Lösung wird durch allmähliche Zugabe von Calciumcarbonat auf pH 3, durch Zugabe von verdünnter Natriumlauge auf pH

5,5 gestellt, vom ausgefallenen Calciumsulfat durch Filtration befreit. Nach Waschen des Gipses mit Wasser dampft man die vereinigten roten Filtrate ein und erhält so einen Farbstoff der Formel

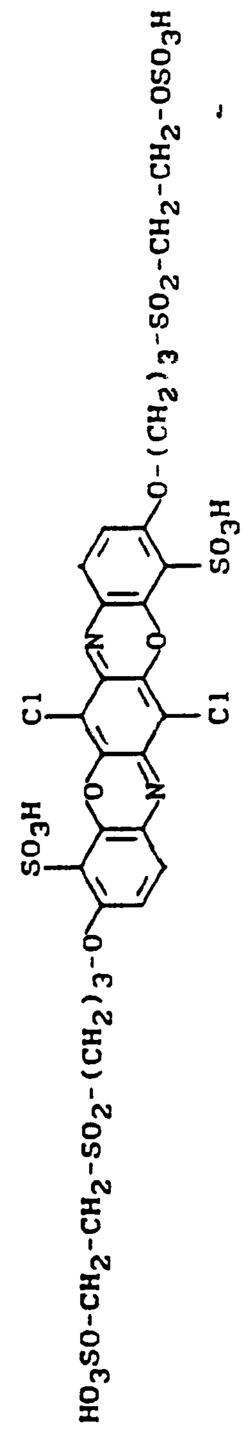

der Cellulosefasern in blaustichig roten Tönen mit guten Echtheiten färbt.

$\lambda_{max}$ = 538 nm, 505 nm in Wasser.

Beispiel 2

50,0 g β-(4-Aminophenoxy)-ethyl-β'-hydroxyethyl-sulfon werden in 500 ml Wasser und 75 ml Isopropanol suspendiert. Man setzt 25,1 g 2,3,5,6-Tetrachlorbenzochinon zu der Suspension, heizt den Ansatz auf 40° und hält den pH-Wert im Reaktionsgemisch mit 2n Sodalösung beständig auf 6,0-6,2. Wenn die Kondensation nach

etwa 5 Stunden beendet ist, saugt man die ausgefallenen hellbraunen Nädelchen ab, wäscht den Filterkuchen mit 1l Wasser und 500 ml Methanol und trocknet ihn bei 60° im Umluftschrank. Das erhaltene Produkt entspricht der Formel

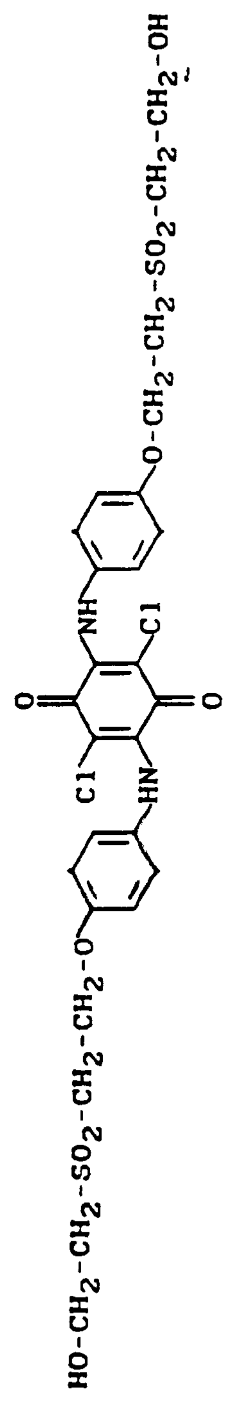

30,0 g des erhaltenen Diarylamino-dichlorchinons werden in 90 ml 20 %iges und 15 ml 65 %iges Oleum bei -5 bis 0° in 2 Stunden gleichmäßig eingetragen. Man steigert die Temperatur auf 20° und rührt einige zeit nach, bis eine chromatographische Probe keine Veränderung mehr anzeigt und in das Molekül neben 2 Sulfatogruppen außerdem 2 Sulfonsäuregruppen eingetreten sind. Man erwärmt den Ansatz auf 30° und trägt im Laufe einer Stunde 24,5 g Kaliumperoxodisulfat ein, hält dabei die Temperatur zwischen 33 und 35° und rührt nach dem Eintragen noch 15-30 Minuten bis zur vollständigen Reaktion weiter. Die blaue Lösung wird in 800 g Eis eingerührt und der Farbstoff durch Eintragen von 150 g Kaliumchlorid ausgesalzen.

Nach einigen Nachrühren wird die Fällung abgesaugt, der Filterkuchen ein- bis zweimal mit 25 %iger Kaliumchloridlösung abgedeckt und anschließend in 75 ml Wasser angeschlagen bzw. gelöst. Man gibt zu der Lösung 2,7 g sekundäres Natriumphosphat und stellt anschließend mit verdünnter Natronlauge oder Sodalösung den pH-Wert auf 5,0-5,5. Aus der Lösung kann der Farbstoff durch Eindampfen oder nach weiterem Verdünnen durch Sprühtrocknung isoliert werden. Er entspricht in Form der freien Säure der Formel

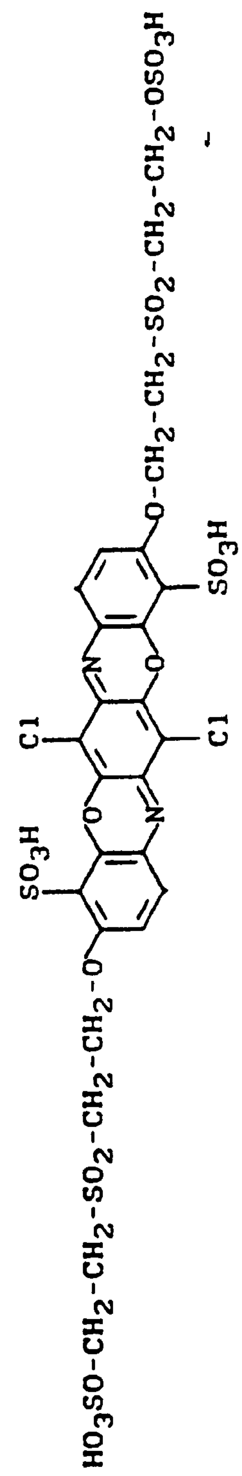

und färbt Wolle oder synthetisches Polyamid in farbstarken, echten Rottönen.

$\lambda_{max}$ = 534 nm, 500 nm in Wasser.

Den gleichen Farbstoff erhält man, wenn man an Stelle des β-(4-Aminophenoxy)-ethyl-β'-hydroxyethylsulfons das β-(4-Aminophenoxy)-ethyl-β'-sulfatoethyl-sulfon des Beispiel 6 oder das β'-(4-Amino-2-sulfophenoxy)-ethyl-β'-sulfatoethylsulfon des Beispiels 17 einsetzt.

Weitere Farbstoffe der allgemeinen Formel I erhält man, wenn man die in der linken Spalte der Tabelle aufgeführten Aminophenoxyalkyl-β'-hydroxyethylsulfone bzw. entsprechende β'-sulfatoethylsulfone oder Amino-sulfophenoxy-alkyl-β-sulfatoethylsulfone mit den Benzochinonen der rechten Spalte kondensiert, die Kondensationsprodukte gegebenenfalls in Schwefelsäure oder Oleum sulfatiert bzw. sulfoniert und sulfatiert und anschließend oxydativ zu Triphendioxazinen, wie gezeigt, ringschließt.

Tabelle

| Nr. | Sulfon | Benzochinon |
|---|---|---|
| 1 | 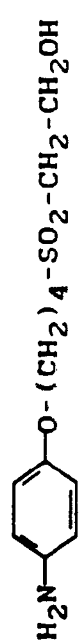 | 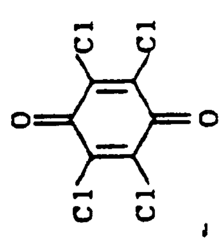 |
| 2 | 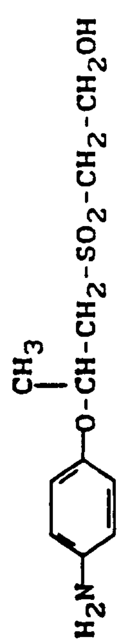 | " |
| 3 | 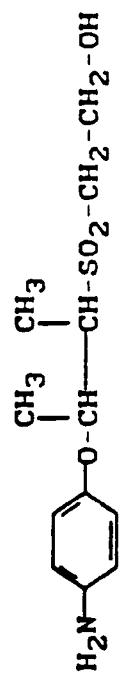 | " |
| 4 | 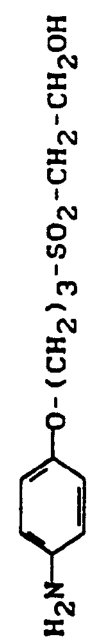 | 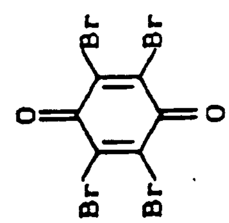 |

<u>Tabelle</u> (Fortsetzung)

| Nr. | Sulfon | Benzochinon |
|---|---|---|
| 5 | 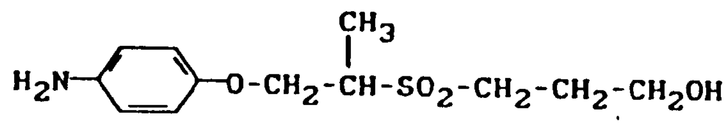 | 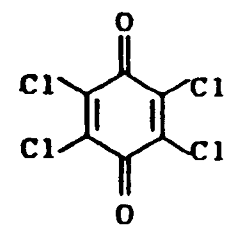 |
| 6 | 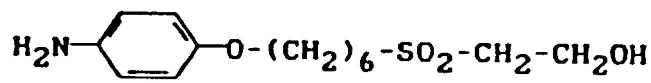 | " |
| 7 | 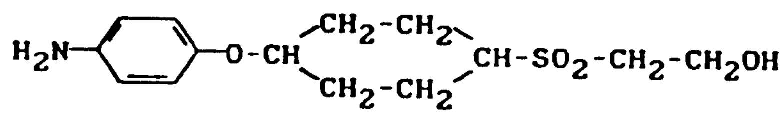 | " |
| 8 | 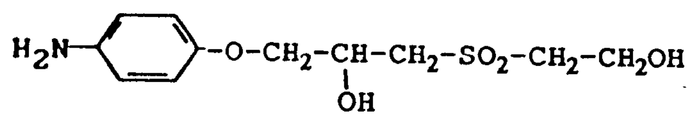 | " |

<u>T a b e l l e</u> (Fortsetzung)

| Nr. | Sulfon | Benzochinon |
|---|---|---|
| 9 | 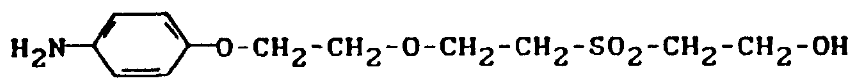 | 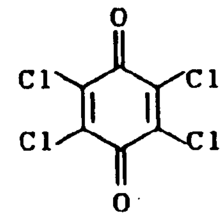 |
| 10 | 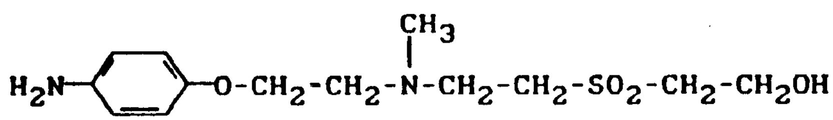 | " |
| 11 | 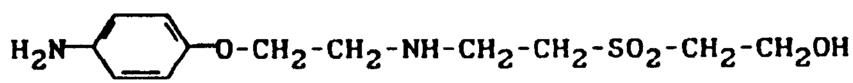 | " |
| 12 | 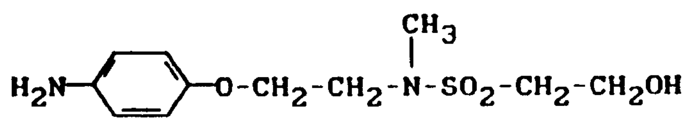 | " |
| 13 | 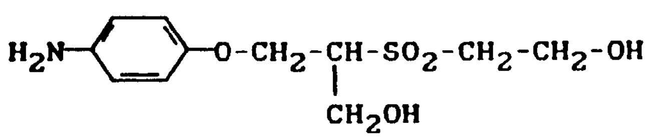 | " |

T a b e l l e (Fortsetzung)

| Nr. | Sulfon | Benzochinon |
|---|---|---|
| 14 | 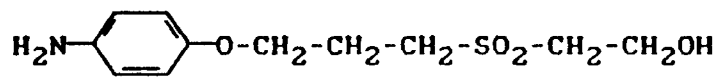 | 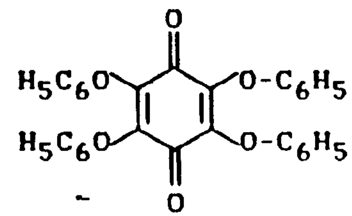 |
| 15 | 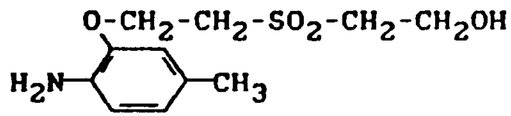 | " |
| 16 | 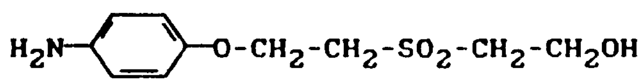 | 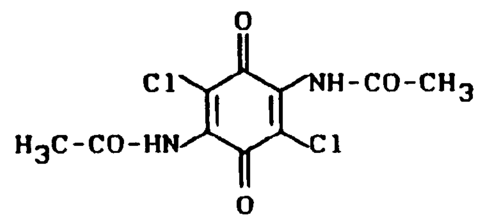 |
| 17 | " | 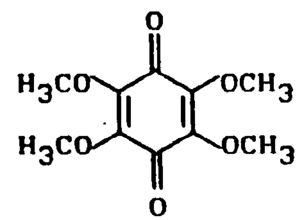 |

T a b e l l e (Fortsetzung)

| Nr. | Sulfon | Benzochinon |
|---|---|---|
| 18 | 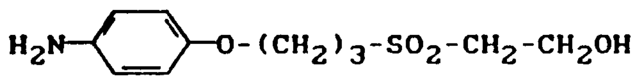 | 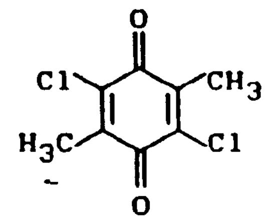 |
| 19 | " | 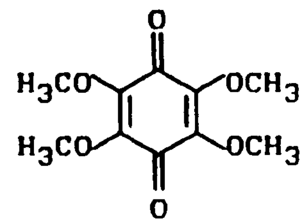 |
| 20 | " | 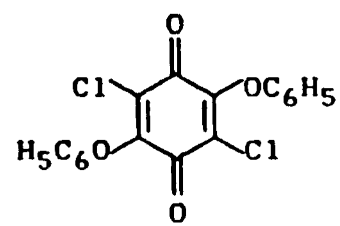 |

**Tabelle** (Fortsetzung)

| Nr. | Sulfon | Benzochinon |
|---|---|---|
| 21 | 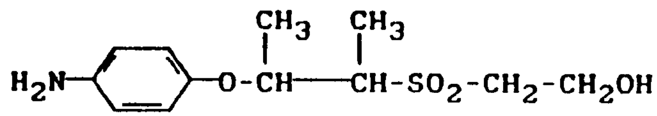 | 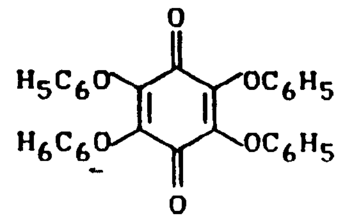 |
| 22 | 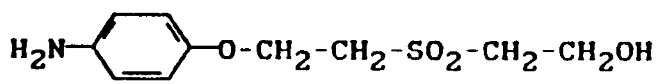 | 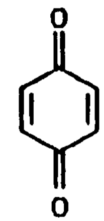 |
| 23 | 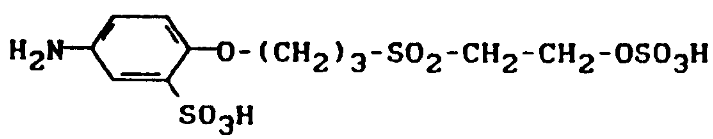 | 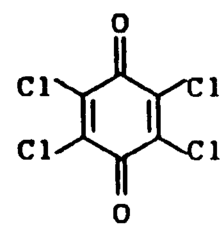 |

T a b e l l e (Fortsetzung)

| Nr. | Sulfon | Benzochinon |
|---|---|---|
| 24 | 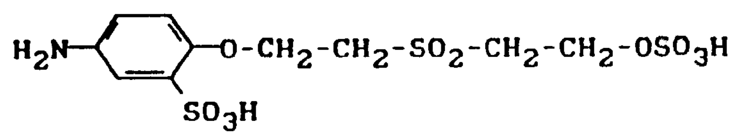 | 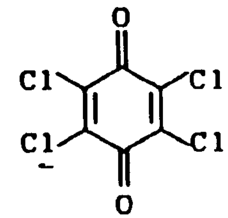 |
| 25 | 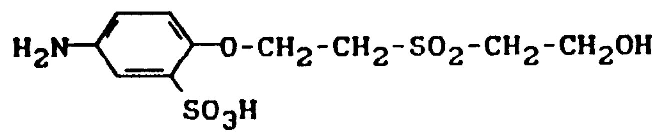 | " |
| 26 | 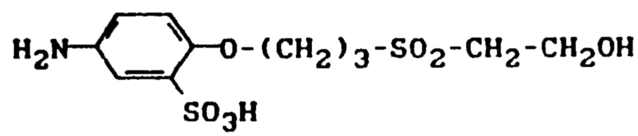 | " |
| 27 | 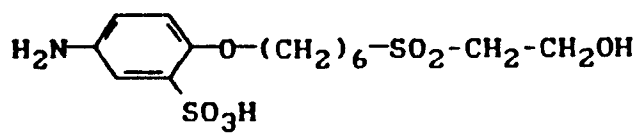 | " |
| 28 | 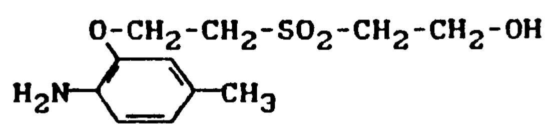 | " |

**T a b e l l e** (Fortsetzung)

| Nr. | Sulfon | Benzochinon |
|---|---|---|
| 29 | 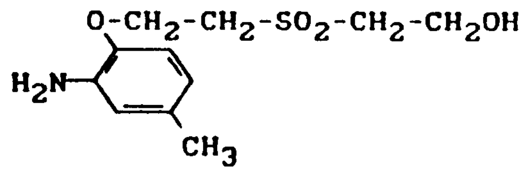 | 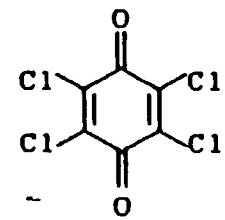 |
| 30 | 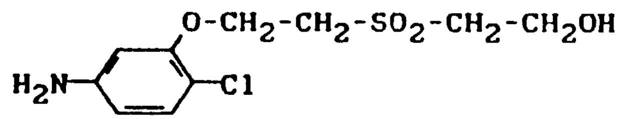 | " |
| 31 | 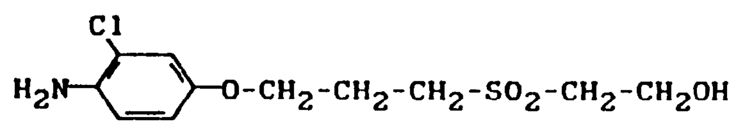 | " |
| 32 | 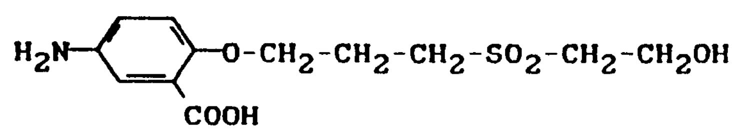 | " |

T a b e l l e (Fortsetzung)

| Nr. | Sulfon | Benzochinon |
| --- | --- | --- |
| 33 | 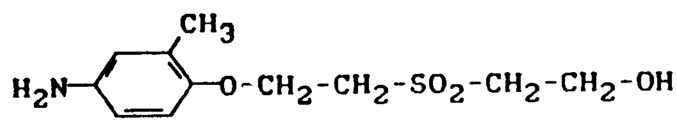 | 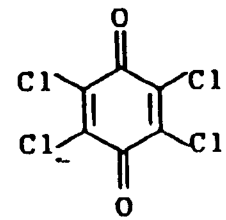 |
| 34 | 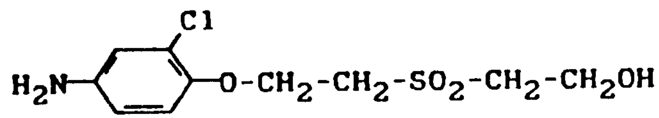 | " |
| 35 | 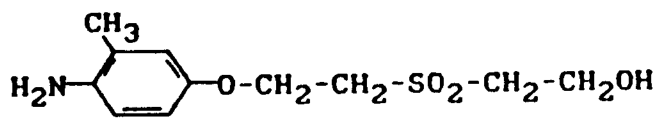 | " |
| 36 | 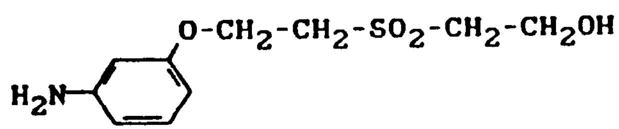 | " |

Tabelle (Fortsetzung)

| Nr. | Sulfon | Benzochinon |
|---|---|---|
| 37 | 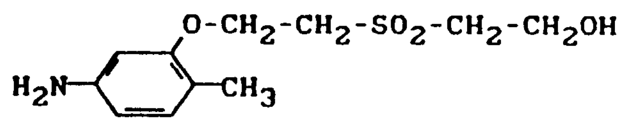 | 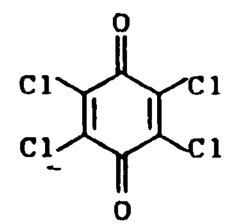 |
| 38 | 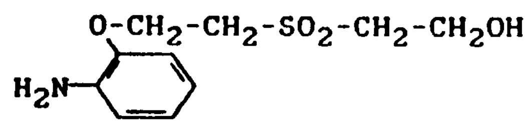 | " |
| 39 | 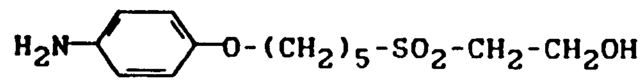 | " |
| 40 | 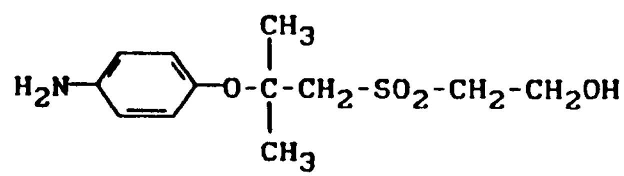 | " |

T a b e l l e (Fortsetzung)

| Nr. | Sulfon | Benzochinon |
|---|---|---|
| 41 | 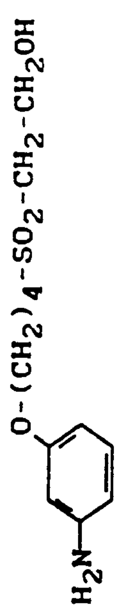 | 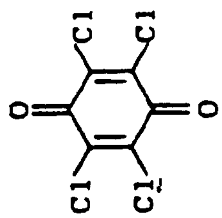 |
| 42 | 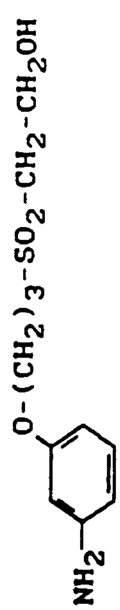 | " |

Beispiel 3

Das eingangs des Beispiels 2 eingesetzte β-(4-Aminophenoxy)-ethyl-β'-hydroxyethyl-sulfon läßt sich auf folgende Weise herstellen:

100 g 1-(2-Hydroxyethoxy)-4-nitrobenzol (hergestellt durch Umsetzung von 4-Nitrophenol mit Ethylenoxid) werden in 300 ml Thionylchlorid eingetragen. Man setzt 1 ml Dimethylformamid zu und erwärmt 1 1/2 Stunden am Rückfluß. Die erhaltene Lösung wird nach Abkühlen in 2 kg Eis-Wasser so eingerührt, daß die Temperatur 0-5° nicht überschreitet. Der weiße Niederschlag wird abgesaugt, mit Wasser neutral gewaschen und im Vakuum getrocknet.

42 g des hergestellten 1-(2-Chlorethoxy)-4-nitrobenzols werden in 100 ml Acetonitril mit 17,8 g 2-Mercaptoethanol und 33 g Kaliumcarbonat 6 Stunden am Rückfluß erhitzt, bis der Austausch des Chloratoms gegen den Mercaptoethanol-Rest vollständig ist.

Gießt man den Ansatz in die zehnfache Menge Wasser, so scheidet sich ein gelbes Öl in praktisch quantitativer Ausbeute ab, das sorgfältig mit Wasser ausgewaschen werden kann. Das Produkt entspricht der Formel

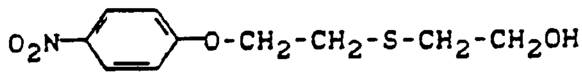

$^1$H-NMR in $D_6$-DMSO (TMS als innerer Standard)

δ = 2,68 ppm (2H, t)
δ = 2,94 ppm (2H, t)
δ = 3,54-3,61 ppm (2H, q)
δ = 4,27 ppm (2H, t)
δ = 4,82 ppm (2H, m)
δ = 7,13 ppm (2H, m)
δ = 8,16 ppm (2H, m)

Für die weitere Oxidation ist es nicht erforderlich, das Sulfid zu isolieren.

Man kann nach der obigen Austausch-Reaktion den Ansatz mit 250 ml Wasser verdünnen. Man erhält so ein gelbliches Zweiphasen-Gemisch, dem man eine neutralisierte Lösung von 0,5 g Wolframsäure in 10 ml Wasser und 5,0 g Natriumacetat zusetzt. Man stellt in der Mischung durch Zugabe von Essigsäure einen pH-Wert von 6,0 ein und läßt dann bei 45° 40,5 g 35 %iges Wasserstoffperoxid in einer Stunde zutropfen. Danach erwärmt man die Mischung 3 Stunden auf 60°, setzt dann erforderlichenfalls noch 4,0 g 35 %iges Wasserstoffperoxid nach und hält die Temperatur bis zur Beendigung der Oxidation auf 60°.

Aus dem erhaltenen Zweiphasen-Gemisch destilliert man das Acetonitril unter vermindertem Druck ab, worauf das Sulfon der Formel

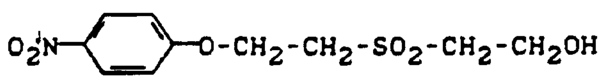

auskristallisiert.

Fp.: 93-94° aus Methylenchlorid/n-Hexan 10:2

$^1$H-NMR in $D_6$-DMSO (TMS als innerer Standard).

δ = 3,36 ppm (2H, t)
δ = 3,63 ppm (2H, t)
δ = 3,84-3,91 ppm (2H, q)
δ = 4,53 ppm (2H, t)
δ = 5,21 ppm (1H, t)
δ = 7,19 ppm (2H, m)
δ = 8,21 ppm (2H, m)

80 g obiges β-(4-Nitrophenoxy)-ethyl-β'-hydroxyethyl-sulfon werden im Autoklaven in 240 ml Methanol nach Zusatz von 4 g Raney-Nickel unter einen Wasserstoffdruck von 50-70 bar gesetzt. Man erwärmt den Autoklaven in 1-2 Stunden auf 60° und hält solange den Wasserstoffdruck aufrecht, bis die Reaktion beendet ist. Der Autoklaveninhalt wird mit 200 ml Methanol versetzt und die Suspension siedend heiß vom Nickel geklärt. Aus dem Filtrat kristallisiert beim Abkühlen das Amin der Formel,

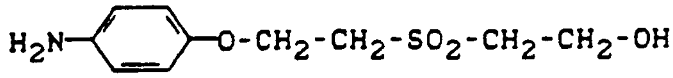

das aus der gekühlten Mischung abgesaugt wird und mit wenig kaltem Methanol gewaschen werden kann.

Fp.: 114-115°.

$^1$H-NMR in $D_6$-DMSO (TMS als innerer Standard).

δ = 3,30 ppm (2H, t)
δ = 3,54 ppm (2H, t)
δ = 3,78-3,85 ppm (2H, q)
δ = 4,18 ppm (2H, t)
δ = 4,65 ppm (2H, s)
δ = 5,14 ppm (1H, t)
δ = 6,49 ppm (2H, m)
δ = 6,66 ppm (2H, m)

An Stelle des oben eingesetzten 1-(2-Chlorethoxy)-4-nitrobenzols kann man auch 1-(2-Bromethoxy)-4-nitrobenzol, p-Toluolsulfonsäure-β-(4-nitrophenoxy)-ethylester analog mit 2-Mercaptoethanol zu β-(4-Nitrophenoxy)-ethyl-β'-hydroxyethylsulfid umsetzen.

Setzt man statt der oben genannten 2-(4-Nitrophenoxy)-ethylhalogenide oder -sulfonsäureester entsprechende 2-(4-Acetaminophenoxy)-ethylhalogenide oder -sulfonsäureester mit 2-Mercaptoethanol um, oxydiert anschließend mit Wasserstoffperoxid in Wasser oder Eisessig und verseift die Acetylaminogruppe in heißer verdünnter Salzsäure, so erhält man ebenfalls p-(4-Aminophenoxy)-ethyl-β'-hydroxyethylsulfon.

Beispiel 4

42,0 g des nach Beispiel 3 hergestellten 1-(2-Chlorethoxy)-4-nitrobenzols werden mit 21,3 g 2-Mercaptoethanol und 14,1 g pulverisiertem Kaliumhydroxid in 160 ml Ethanol unter Stickstoffatmosphäre mehrere Stunden am Rückfluß gekocht, bis eine chromatographische Probe den vollständigen Austausch des Chloratoms im Edukt gegen den β-Hydroxyethylmercaptid-Rest anzeigt.

Man verdünnt die erhaltene Suspension mit 320 ml Wasser, versetzt die erhaltene Emulsion mit einer neutralisierten Lösung von 0,5 g Wolframsäure in 10 ml Wasser und stellt nach Zugabe von 2,5 g Natriumacetat den pH-Wert der Mischung mit Essigsäure auf 5,5. Zur Oxidation des Sulfids tropft man bei 50-60° 55 ml 35 %iges Wasserstoffperoxid im Laufe von einer Stunde und hält anschließend die Temperatur noch 3-4 Stunden auf 60°, bis die Oxidation des als Zwischenprodukt entstehenden Sulfoxids zum Sulfon vollständig ist.

Aus der klaren Lösung wird das Ethanol abdestilliert. Die anfallende Emulsion scheidet auf Animpfen bei Raumtemperatur das Sulfon der Formel

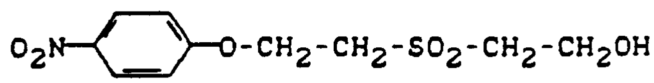

in Kristallen ab, die nach Absaugen und Waschen mit Wasser gegebenenfalls getrocknet werden.

Katalytische Reduktion mit Wasserstoff und Raney-Nickel nach den Angaben des Beispiels 3 führt zur entsprechenden Aminoverbindung.

Beispiel 5

30,0 g 1-(2-Hydroxyethoxy)-4-nitrobenzol werden in 60 ml Pyridin gelöst. Man läßt 18,9 g Methansulfonsäurechlorid in 15 Minuten bei 10-15° zutropfen und anschließend die Temperatur auf 20° ansteigen. Nach 3 Stunden ist die Umsetzung beendet. Zu der entstandenen Lösung werden 60 ml Wasser zugetropft, worauf der Methansulfonsäureester in schönen Nadeln kristallisiert. Man saugt die Kristalle ab, wäscht sie mit Wasser und erhält ein Produkt der Formel

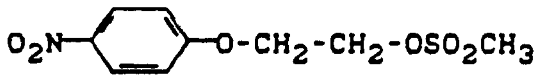

vom Fp.: 70-71,5°.

11,7 g 2-Mercaptoethanol werden in 150 ml 1n Natronlauge gelöst. Die Lösung wird mit 30,0 g pulverisiertem Methansulfonsäureester der obigen Formel versetzt und die erhaltene Suspension unter Stickstoffatmosphäre gesetzt. Man erhitzt mehrere Stunden auf 70°, bis der Austausch des Methansulfonsäureesters vollständig ist. Es hat sich ein gelbes Öl der Formel

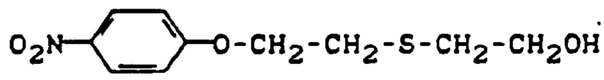

gebildet, das mit dem Sulfid des Beispiels 3 identisch ist. Das Produkt wird abgetrennt, mit verdünnter Sodalösung und Wasser gewaschen und anschließend in wäßriger Emulsion mit 23 g 35%igem Wasserstoffperoxid in der im Beispiel 3 beschriebenen Weise unter Zusatz von katalytischen Mengen Wolframsäure zu dem dort beschriebenen Sulfon oxidiert.

Beispiel 6

35,0 g 2-(4-Aminophenoxyethyl)-2-hydroxyethylsulfon des Beispiels 3 werden in 70 ml 96 %ige Schwefelsäure bei 0-5° eingetragen. Man rührt 2 Stunden und trägt die erhaltene Lösung in 420 g Eis ein. Die kristalline Fällung wird abgesaugt, mit 25 %iger Natriumchloridlösung bis zur Sulfatfreiheit gewaschen und bei 50° im Vakuum getrocknet. Das erhaltene Produkt entspricht der Formel

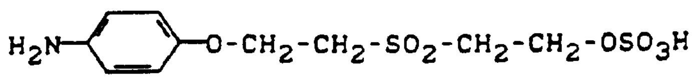

bzw. dessen innerem Salz.

$^1$H-NMR-Analyse in $D_6$-DMSO (TMS als innerer Standard).

$\delta$ = 3,51 ppm (2H, t)
$\delta$ = 3,62 ppm (2H, t)
$\delta$ = 4,12 ppm (2H, t)
$\delta$ = 4,35 ppm (2H, t)
$\delta$ = 7,04-7,08 ppm (2H, d)
$\delta$ = 7,27-7,31 ppm (2H, d)
$\delta$ = 10,1 ppm (3H, s)

Beispiel 7

Das eingangs des Beispiels 1 eingesetzte 3-(4-Aminophenoxy)-propyl-2-hydroxyethylsulfon läßt sich auf folgende Weise gewinnen:

111 g 1-(3-Hydroxypropoxy)-4-nitrobenzol (hergestellt beispielsweise aus 4-Nitrofluorbenzol und 1,3-Propandiol oder aus 4-Nitrophenol und 3-Brom- oder 3-Chlor-1-propanol) werden in 113 ml Pyridin gelöst. Dazu läßt man eine Lösung von 102 g p-Toluolsulfochlorid in 107 ml Pyridin in 30 Minuten bei 5° beginnend zutropfen und die Temperatur durch Reaktions- und Kristallisationswärme auf 20° ansteigen. Man kühlt den Kristallbrei auf 0° ab, saugt ihn ab, wäscht die Kristalle mit 400 ml eiskaltem Methanol, anschließend mit Wasser und trocknet sie bei 50° im Vakuum.

Fp.: 136-139°.

85 g des erhaltenen Toluolsulfonsäureesters werden in 240 ml Acetonitril mit 21, 2 g 2-Mercaptoethanol und 38,5 g Kaliumcarbonat 2,5 Stunden am Rückfluß bei 84° erhitzt. Nach Eingießen in 1l Wasser scheidet sich ein gelbes Öl ab, das der Formel

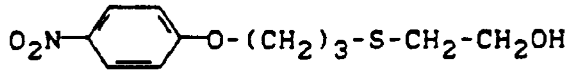

entspricht und in Methylenchlorid aufgenommen werden kann. nach Trocknen der organischen Phase über Natriumsulfat und Abdestillieren des Methylenchlorids kann man das Sulfid als bei 43-46° erstarrendes Öl erhalten.

$^1$H-NMR in $D_6$-DMSO (TMS als innerer Standard).

δ = 1,97-2,08 ppm (2H, m)
δ = 2,58-2,73 ppm (4H, m)
δ = 3,55 ppm (2H, m)
δ = 4,20 ppm (2H, t)
δ = 4,81 ppm (1H, s)
δ = 7,11-7,15 ppm (2H, d)
δ = 8,16-8,20 ppm (2H, d)

Statt des Tosylesters kann man analog Beispiel 3 auch 3-(4-Nitrophenoxy)-1-propylchlorid oder -1-propylbromid, die man aus 4-Nitrophenol und den 1,3-Dihalogenpropanen herstellt, mit 2-Mercaptoethanol zu obigen Sulfid umsetzen.

Zur Oxidation des Sulfids verfährt man im allgemeinen so, daß man das nach der Mercaptid-Austausch-Reaktion anfallende Öl-Wasser-Gemisch analog Beispiel 3 direkt unter Wolframsäurekatalyse mit 50,0 g 35 %igem Wasserstoffperoxid bei 60° oxidiert. Nach Abkühlen wird das kristallin anfallende Sulfon der Formel

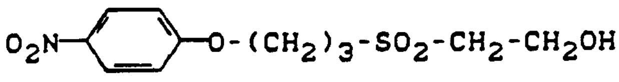

isoliert.

Fp.: 104-106°.
$^1$H-NMR in $D_6$-DMSO (TMS als innerer Standard).
δ = 2,15-2,27 ppm (2H, m)
δ = 3,26-3,38 ppm (4H, m)
δ = 3,78-3,85 ppm (2H, q)
δ = 4,23 ppm (2H, t)
δ = 5,15 ppm (1H, t)
δ = 7,09-7,13 ppm (2H, d)
δ = 8,15-8,19 ppm (2H, d)

Eine weitere Variante zur Herstellung des obigen Sulfons besteht darin, 61 g des oben erhaltenen von Wasser befreiten 8-(4-Nitrophenoxy)-propyl-β-hydroxyethylsulfids in 122 ml Eisessig zu lösen und mit 47 g 35 %igem $H_2O_2$ bei 30° beginnend zu oxidieren und zur Vervollständigung der Oxidation anschließend mehrere Stunden auf 40° zu erwärmen, bis das als Zwischenprodukt gebildete Sulfoxid chromatographiert nicht mehr nachweisbar ist. Die Isolierung des Sulfons erfolgt durch Verdünnen des Ansatzes mit 125 ml Wasser, Absaugen und Waschen mit Wasser, wodurch ein mit dem vorstehend beschriebenen Sulfon identisches Produkt vom Fp.: 104-106° erhalten wird.

50 g des obigen Nitrosulfons weden im Autoklaven in 200 ml Methanol nach Zusatz von 2,5 g Raney-Nickel bei 60° unter einem Wasserstoffdruck von 60 bar reduziert. Wenn kein Wasserstoff mehr aufgenommen wird, verdünnt man den Autoklaveninhalt mit 200 ml Methanol und 100 ml Wasser und saugt siedend heiß vom Nickel ab. Aus dem Filtrat kristallisiert die Aminoverbindung der Formel

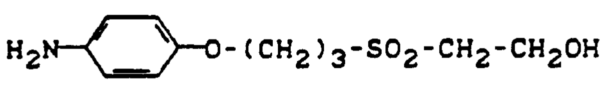

in schönen Kristallen vom Fp.: 136,5-138°.

$^1$H-NMR in $D_6$-DMSO (TMS als innerer Standard).
δ = 2,02-2,14 ppm (2H, m)
δ = 3,23-3,29 ppm (4H, m)
δ = 3,77-3,84 ppm (2H, q)
δ = 3,91 ppm (2H, t)
δ = 4,60 ppm (2H, s)
δ = 5,14 ppm (1H, t)
δ = 6,47-6,51 ppm (2H, d)
δ = 6,62-6,66 ppm (2H, d)

Beispiel 8

50 g β-(4-Nitrophenoxy)-β'-hydroxyethylsulfid werden in 300 ml 20 %iger Salzsäure verrührt. In die entstandene Emulsion leitet man einen langsamen Chlorstrom ein und führt durch äußere Kühlung die entstehende Reaktionswärme so ab, daß die Temperatur bei 30° gehalten wird.

Bald scheiden sich aus der Emulsion schöne Kristalle ab. Wenn kein Chlor mehr verbraucht wird, prüft man chromatographisch auf Vollständigkeit der Oxidation. Das erhaltene Produkt wird abgesaugt und mit Wasser chloridfrei gewaschen. Es entspricht der Formel

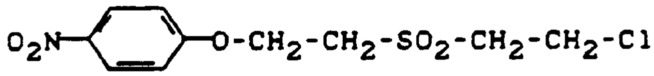

Fp.: 85-86°.

$^1$H-NMR in $D_6$-DMSO (TMS als innerer Standard).

δ = 3,73-3,82 ppm (4H, m)
δ = 3,99 ppm (2H, t)
δ = 3,53 ppm (2H, t)
δ = 7,17-7,21 ppm (2H, d)
δ = 8,19-8,23 ppm (2H, d)

Beispiel 9

75,0 g 4-Nitrophenol werden in 750 ml Wasser und 130 ml 4n Natronlauge bei pH 8,5 und 80° gelöst. Zu der Lösung tropft man im Laufe von 8 Stunden 62,5 g Propylenoxid und hält dabei den pH-Wert durch Zutropfen von 2n Schwefelsäure auf 8,5. Ist nach Aufrechterhalten von pH 8,5 und 80° über Nacht noch wenig Nitrophenol vorhanden, so setzt man gegebenenfalls noch 16 g Propylenoxid nach und hält die Reaktionsbedingungen, bis kein Edukt mehr nachweisbar ist. Als Produkt hat sich ein Öl aus der wäßrigen Phase abgeschieden, daß abgetrennt und getrocknet wird. Das erhaltene Produkt stellt ein Isomerengemisch folgender Substanzen dar.

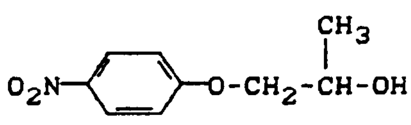

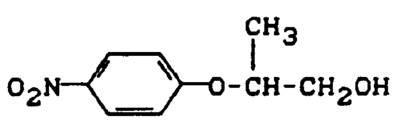

72,0 g des erhaltenen Öles werden in 73 ml Pyridin gelöst. Man läßt bei 10-15° eine Lösung von 69,7 g p-Toluolsulfochlorid in 73 ml Pyridin unter Kühlung zutropfen und rührt anschließend bei 0-5° über Nacht. Aus der zunächst klaren Lösung fallen Kristalle aus, die in der Kälte abgesaugt, mit 200 ml eiskaltem Methanol und anschließend mit Wasser gewaschen werden. Man erhält 56,5 g des reinen Isomeren der Formel

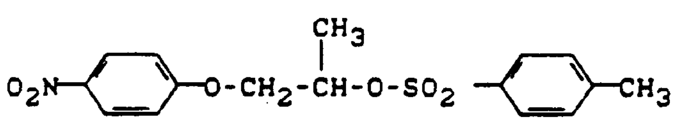

vom Fp.: 84-86°.

$^1$H-NMR in $D_6$-DMSO (TMSO als innerer Standard).

δ = 1,41 ppm (3H, d)
δ = 2,43 ppm (3H, s)

δ = 4,01-4,11 ppm (2H, m)
δ = 4,82-4,93 ppm (1H, m)
δ = 6,73-6,76 ppm (2H, d)
δ = 7,29-7,33 ppm (2H, d)
δ = 7,73-7,76 ppm (2H, d)
δ = 8,07-8,11 ppm (2H, d)

Verdünnt man die vereinigte Mutterlauge und Methanol-Wäsche nach der Abtrennung des obigen Toluolsulfonsäureesters mit 1l Wasser, so lassen sich aus der Emulsion durch Extrahieren mit Methylenchlorid, Trocknen des Extraktes, Verdampfen des Lösungsmittels und Versetzen des Rückstandes mit 100 ml Methanol nach Isolierung der ausgefallenen Kristalle noch 50,8 g eines Isomeren-Gemisches der Formeln

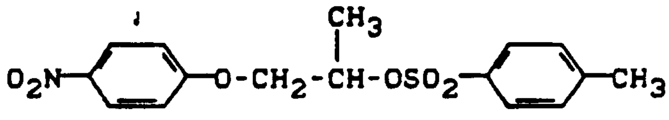

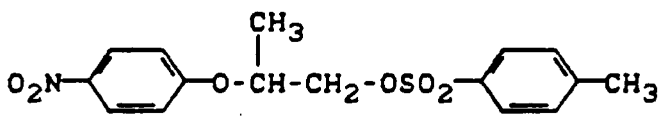

gewinnen, das zwischen 68-72° schmilzt.

56,0 g obiger isomerenfreier Toluolsulfonsäureester vom Fp.: 84-86° werden in 150 ml Acetonitril unter Stickstoffatmosphäre mit 13,0 g 2-Mercaptoethanol und 23 g Kaliumcarbonat mehrere Stunden zum Rückfluß erhitzt, bis der Austausch des Tosylat- gegen den β-Hydroxyethylmercaptid-Rest vollständig ist. Nach Eingießen des Ansatzes in 750 ml Wasser scheidet sich ein gelbes Öl folgender Formel ab:

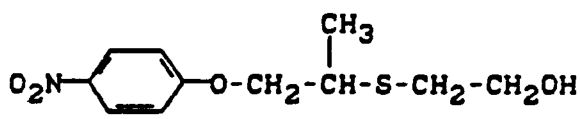

Zu der erhaltenen Öl-Wasser-Mischung setzt man eine neutralisierte Lösung von 0,3 g Wolframsäure in 10 ml Wasser und 3 g Natriumacetat, stellt den pH-Wert im Gemisch mit Essigsäure auf 6,0 und oxidiert mit 31,6 g 35 %igem Wasserperoxid bei 45° beginnend bis 60°. Man erhält das Sulfon der Formel

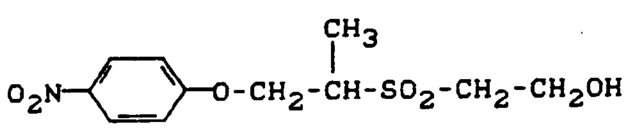

ebenfalls als Öl, das abgetrennt, in 150 ml Methanol gelöst und mit 3 g Raney-Nickel unter einem Wasserstoffdruck von 60 bar bei 50° reduziert wird. Nach chromatographischer Prüfung auf Vollständigkeit der Reduktion wird das Methanol durch Vakuumdestillation im Rotationsverdampfer entfernt.

Es hinterbleibt das Amin der Formel

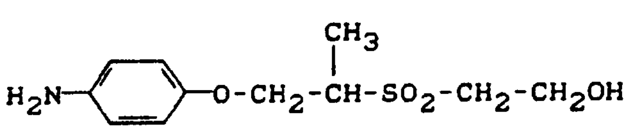

wiederum als Öl.

Führt man die gleiche Reaktonsfolge statt mit dem abgetrennten isomerenfreien Toluolsulfonsäureester direkt mit dem anfallenden Toluolsulfonsäureester-Isomeren-Gemisch durch, so erhält man nach dem Mercaptid-Austausch, der Oxidation des Sulfid-Gemisches und der Reduktion der Nitrosulfon-Isomeren wiederum als Öl ein Isomeren-Gemisch, in dem die Komponenten

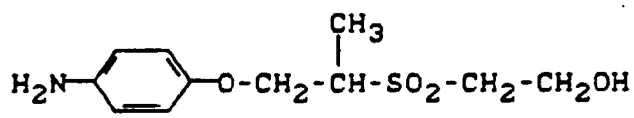

und

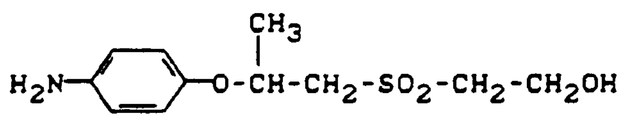

etwa im Verhältnis 4:1 vorliegen.

Beispiel 11

60,0 g 4-Nitrophenol werden in 600 ml Wasser und 107 ml 2n Natronlauge bei 80° und pH 8,5 gelöst. Man läßt bei 80° im Laufe von 24 Stunden 94 g trans-2,3-Butylenoxid zutropfen und hält gleichzeitig den pH-Wert mit 2n Schwefelsäure auf 8,5. Nach beendeter Umsetzung hat sich aus der wäßrigen Phase ein Öl abgeschieden, das isoliert und getrocknet wird.

$^1$H-NMR in $D_6$-DMSO (TMS als innerer Standard).
δ = 1,12-1,15 ppm (3H, d)
δ = 1,23-1,25 ppm (3H, d)
δ = 3,72-3,83 ppm (1H, m)
δ = 4,40-4,49 ppm (1H, m)
δ = 4,88-4,90 ppm (1H, d)
δ = 7,09-7,13 ppm (2H, d)
δ = 8,12-8,16 ppm (2H, d)

75,0 g des erhaltenen Öles werden in 75 ml Pyridin gelöst. Dazu läßt man eine Lösung von 67,7 g p-Toluolsufochlorid in 67 ml Pyridin zutropfen und hält die Temperatur auf 10°. Nach mehrstündigem Rühren bei 0-5° saugt man die ausgefallenen Kristalle bei 0° ab, wäscht sie mit 200 ml eiskaltem Methanol und dann mit Wasser und trocknet sie bei 50° im Vakuum.

Fp.: 116-118°.
$^1$H-NMR in $D_6$-DMSO (TMS als innerer Standard).
δ = 1,27-1,30 ppm (3H, d)
δ = 1,36-1,38 ppm (3H, d)
δ = 2,42 ppm (3H, s)
δ = 4,48-4,57 ppm (1H, m)
δ = 4,67-4,77 ppm (1H, m)
δ = 6,74-6,78 ppm (2H, d)
δ = 7,27-7,31 ppm (2H, d)
δ = 7,70-7,74 ppm (2H, d)
δ = 8,07-8,11 ppm (2H, d)

100 g des erhaltenen Toluolsulfonsäureesters werden in 300 ml Acetonitril mit 24 g 2-Mercaptoethanol und 43,5 g Kaliumcarbonat unter Stickstoffatmosphäre 24 Stunden am Rückfluß erhitzt, bis das Edukt verschwunden, der Austausch gegen den β-Hydroxyethylmercaptid-Rest vollständig ist. Man gießt man den Ansatz in 750 ml Wasser, worauf sich das Sulfid der Formel

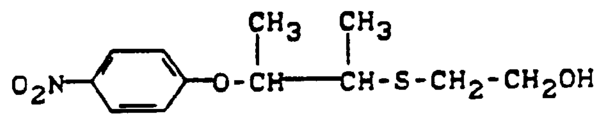

als Öl abscheidet.

Das Öl-Wasser-Gemisch wird mit einer neutralisierten Lösung von 1,0 g Wolframsäure in 20 ml Wasser versetzt und nach Zusatz von 3 g Natriumacetat mit Essigsäure auf den pH-Wert von 6,0 gestellt.

Oxidation mit 58 g 30 %igem Wasserperoxid bei 45-60° analog den Angaben des Beispiels 3 ergibt das Sulfon der Formel

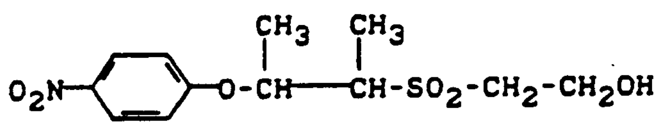

das als Öl anfällt, abgetrennt, mit Wasser gewaschen und zur katalytischen Reduktion in 400 ml Methanol gelöst wird. Man reduziert die Nitroverbindung nach Zusatz von 5 g Raney-Nickel bei 50° unter einem Wasserstoffdruck von 60 bar. Nach Abfiltration vom Nickel wird das Methanol aus dem Filtrat im Vakuum abdestilliert. Als öliger Rückstand verbleibt die Verbindung der Formel

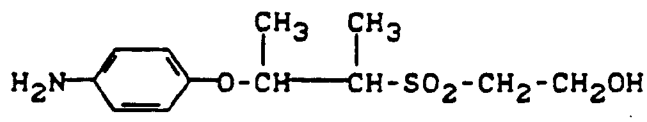

Beispiel 12

Die Verbindung des Beispiels 3 oder 4 der Formel

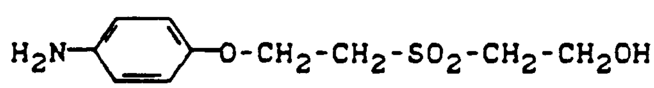

läßt sich auch gewinnen, indem man 45,0 g 4-Nitrofluorbenzol zu einer Mischung von 200 ml Acetonitril, 115 g Bis-(2-hydroxyethyl)-sulfid und 18,0 g pulverisiertem Kaliumhydroxid bei 25° zutropfen läßt und das Reaktionsgemisch 4 Stunden bei 25° rührt. Nach beendeter Umsetzung wird das Gemisch in 2l Wasser gegossen und das ausgefällte rohe β-Hydroxyethyl-β'-(4-nitrophenoxy)-ethyl-sulfid als gelbes Öl abgetrennt und mit Wasser gewaschen.

Es wird in 150 ml Eisessig gelöst und bei 40-45° mit 85 g 35 %igem Wasserstoffperoxid zum Sulfon oxidiert.

Danach ist als Nebenprodukt aus dem symmetrischen Sulfid entstandenes Bis-2-(4-nitrophenoxy)-ethylsulfon in Form von Nadeln ausgefallen. Man trennt das Bisprodukt durch Filtration ab und fällt aus dem Filtrat durch Verdünnen mit Wasser das Nitrosulfon der Formel

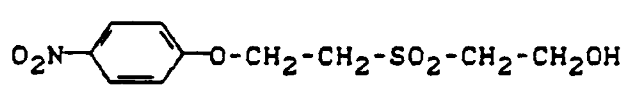

aus, das sich in der im Beispiel 3 geschilderten Weise zu der eingangs erwähnten Aminoverbindung reduzieren läßt.

Beispiel 13

154 g 1-(2-Hydroxyethoxy)-3-nitrobenzol (hergestellt durch Umsetzung von 3-Nitrophenol mit 2-Chloretha-

nol bei 70-80° und pH 8,5 in Wasser) werden in 460 ml Thionylchlorid eingetragen. Nach beendeter Gasentwicklung setzt man 4 ml Dimethylformamid zu und erhitzt 2 Stunden am Rückfluß. Aus dem Reaktionsgemisch wird nun der größte Teil des Thionylchlorids abdestilliert und der verbleibende Rückstand in 2 kg Eis eingetragen. Das in fester Form abgeschiedene Reaktionsprodukt wird zerkleinert, sorgfältig mit Wasser neutral gewaschen und bei 40° im Vakuum getrocknet.

Fp. des Rohproduktes: 57-58°.

126 g des so hergestellten 1-(2-Chlorethoxy)-3-nitrobenzols werden in 630 ml Acetonitril mit 48 ml 2-Mercaptoethanol und 9,9 g Kaliumcarbonat unter Stickstoffatmosphäre auf 70° erwärmt. Nach 5 Stunden setzt man 12 ml 2-Mercaptoethanol und 25 g Kaliumcarbonat nach und führt die Umsetzung unter chromatographische Kontrolle bei 70° zu Ende. Nach beendeter Umsetzung wird der Ansatz mit 2,2 l Wasser verdünnt. Das ausgefallene honiggelbe Öl wird abgetrennt, mit Wasser gewaschen und kann nach Lösen in Methylenchlorid über Natriumsulfat getrocknet werden oder im Vakuum vom Wasser befreit werden.

Statt in Acetonitril kann man die Umsetzung der 2-Chlorethoxyverbindung mit 2-Mercaptoethanol auch in dem gleichen Volumen Ethanol als Lösungsmittel durchführen.

Man erhält ebenfalls die Verbindung

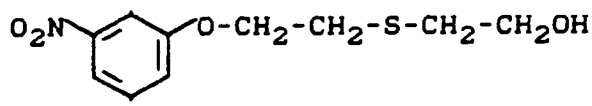

als Öl.

$^1$H-NMR in $D_6$-DMSO (mit TMS als inneren Standard).

δ = 2,71 ppm (2H,t)
δ = 2,96 ppm (2H, t)
δ = 3,57-3,65 ppm (2H, q)
δ = 4,27 ppm (2H, t)
δ = 4,84 ppm (1M, t)
δ = 7,38-7,43 ppm (1H, m)
δ = 7,56 ppm (1H, t)
δ = 7,69 ppm (1H, t)
δ = 7,78-7,83 ppm (1H, m)

140,6 g des oben erhaltenen Öles werden in 600 ml Wasser emulgiert. Nach Zusatz von 7,2 g Natriumacetat wird mit ca. 1,4 ml Eisessig ein pH-Wert von 5,2 eingestellt.

Man setzt eine Katalysatorlösung zu, die durch Lösen von 1,2 g Wolframsäure in 24 ml Wasser und 1,2 ml 50 %iger Natronlauge und Einstellen eines pH-Wertes von 5,2 mittels Eisessig hergestellt worden war.

Im Laufe einer Stunde werden zu dieser Emulsion 104 ml 35 %iges Wasserstoffperoxid bei 55-60° zugetropft. Man hält dann die Temperatur weiter auf 60°, setzt nach 4 Stunden noch 20 ml 35 %iges Wasserstoffperoxid nach und hält weitere 2 Stunden auf Temperatur, bis eine chromatographische Probe kein Sulfoxid-Zwischenprodukt mehr zeigt. Man kühlt auf 5° ab, die entstandene Emulsion scheidet Kristalle ab, die isoliert, zerkleinert und mit eiskaltem Wasser gewaschen werden.

Man erhält ein Produkt der Formel

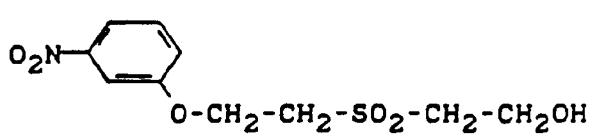

Fp.: 89-91° (nach Umkristallisation aus 5 Volumina Methanol und 2 Volumina Wasser)

$^1$H-NMR in $D_6$-DMSO (mit TMS als innerem Standard)

δ = 3,38 ppm (2H, t)
δ = 3,72 ppm (2H, t)
δ = 3,83-3,90 ppm (2H, q)
δ = 4,52 ppm (2H, t)
δ = 5,19 ppm (1H, t)
δ = 7,43-7,49 ppm (1H, m)
δ = 7,60 ppm (1H, t)

δ = 7,78 ppm (1H, m)
δ = 7,82-7,86 ppm (1H, m)

142 g der erhaltenen Nitroverbindung werden in 600 ml Methanol nach Zusatz von 10 g Raney-Nickel unter einen Wasserstoffdruck von 70 bar gesetzt und die Temperatur auf 60° gesteigert. Man hält den Wasserstoffdruck bis zu beendetem Wasserstoffverbauch konstant, entspannt nach Abkühlung, klärt die erhaltene Suspension vom Raney-Nickel und dampft das Filtrat im Rotationsverdampfer bis zur völligen Verdämpfung des Lösungsmittels ein.

Man erhält das Produkt der Formel

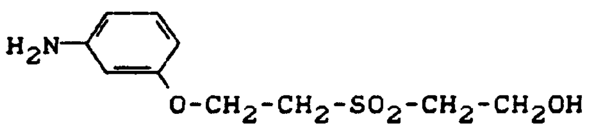

als dickflüssiges Öl, das erst nach mehrtägigem Stehen kristallisiert. Fp.: 70-72° (aus 4 Volumina Isopropanol)

$^1$H-NMR in $D_6$-DMSO (TMS als innerer Standard).
δ = 3,30 ppm (2H, t)
δ = 3,57 ppm (2H, t)
δ = 3,78-3,85 ppm (2H, q)
δ = 4,21 ppm (2H, t)
δ = 5,04 ppm (2H, s)
δ = 5,14 ppm (1H, t)
δ = 6,06-6,16 ppm (3 x 1H, m)
δ = 6,84-6,91 ppm (1H, t)

Beispiel 14

50 g des in Beispiel 13 erhaltenen Öles der Verbindung

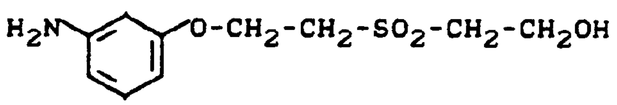

läßt man portionsweise in 125 ml 96 %ige Schwefelsäure bei 0° einlaufen. Man rührt 2 Stunden bei dieser Temperatur nach und trägt die erhaltene Lösung anschließend auf 900 g Eis aus. Es scheiden sich rundliche Kristalle ab. Nach kurzem Nachrühren saugt man eiskalt ab und wäscht das erhaltene Produkt mit Isopropanol oder 25 %iger Natriumchloridlösung schwefelsäurefrei. Es entspricht der Formel

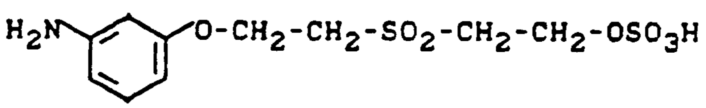

Man trocknet es im Vakuum.

$^1$H-NMR in $D_6$-DMSO (TMS als innerer Standard).
δ = 3,40 ppm (2H, t)
δ = 3,63 ppm (2H, t)
δ = 4,12 ppm (2H, t)
δ = 4,36 ppm (2H, t)
δ = 6,89-6,92 ppm (2x 1H, m?)
δ = 6,99-7,02 ppm (1H, m)
δ = 7,35-7,41 ppm (1H, t)

Beispiel 15

Zu 250 g 2-Nitrophenol in 2500 ml Wasser gibt man etwa 80 ml 50 %ige Natronlauge bis zum pH 8,5.

Zu der erhaltenen Lösung läßt man in 8 Stunden bei 80° 420 ml 2-Chlorethanol portionsweise zulaufen, hält den pH-Wert dabei mit Natronlauge auf 8,5 und heizt unter diesen Bedingungen noch weitere 4 Stunden auf 80°, bis das Edukt praktisch verschwunden ist. Das entstandene Öl des 1-(2-Hydroxyethoxy)-2-nitrobenzols läßt man bei Raumtemperatur absitzen, wäscht es mehrfach mit Wasser und entfernt Wasserspuren durch Erwärmen im Vakuum bis 70°.

280 g des erhaltenen Öles werden in 30 Minuten bei 20-25° unter leichter Kühlung mit 800 ml Thionylchlorid versetzt. Nach Zugabe von 5 ml Dimethylformamid erwärmt man 3 Stunden zum Rückfluß auf etwa 80°. Die erhaltene Lösung wird nach Abkühlen in 5 kg Eis eingerührt, das zunächst erhaltene Öl erstarrt bald zu Kristallbrocken. Die feste Masse wird abgesaugt, zerkleinert und mit Wasser neutral gewaschen und im Vakuum getrocknet.

252 g des erhaltenen 1-(2-Chlorethoxy)-2-nitrobenzols werden in 1000 ml Acetonitril mit 26 ml 2-Mercaptoethanol und 198 g Kaliumcarbonat unter Stickstoffatmosphäre 4 Stunden auf 70° erwärmt. Man setzt dann 24 ml 2-Mercaptoethanol und 50 g Kaliumcarbonat nach und erhitzt noch weitere 4 Stunden bei 80° am Rückfluß. Nach Abkühlung wird der Ansatz mit 4l Wasser verdünnt, das ausgeschiedene Öl abgetrennt, mit Wasser gewaschen und durch Erhitzen im Vakuum bis zuletzt 80° von Wasserresten befreit.

Man kann auch nach Verdünnen des Ansatzes mit Wasser das Gemisch mit Methylenchlorid ausschütteln, die organische Phase mehrmals mit Wasser waschen und über Natriumsulfat trocknen. Nach Abdestillieren des Methylenchlorids zuletzt im Vakuum bis 80° erhält man als Destillationsrückstand wie oben ebenfalls ein Öl.

Das erhaltene Produkt hat die Formel

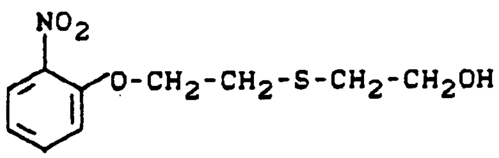

$^1$H-NMR in $D_6$-DMSO (TMS als innerer Standard).

δ = 2,66 ppm (2H, t)
δ = 2,90 ppm (2H, t)
δ = 3,53-3,60 ppm (2H, q)
δ = 4,28 ppm (2H, t)
δ = 4,78 ppm (1H, t)
δ = 7,04-7,11 ppm (1H, m)
δ = 7,31-7,34 ppm (1H, d?)
δ = 7,55-7,63 ppm (1H, m)
δ = 7,78-7,83 ppm (1H, m)

150 g des Ölgs der zuletzt genannten Formel werden in 1000 ml Wasser durch Rühren emulgiert. Man setzt 7,2 g Natriumacetat hinzu, stellt mit Essigsäure den pH-Wert auf 5,2 und versetzt mit einer Katalysatorlösung von 1,2 g Wolframsäure, die in der im Beispiel 13 beschriebenen Weise zubereitet war.

Innerhalb einer Stunde werden bei 55-60° 111 ml 35 %iges Wasserstoffperoxid zugetropft, wobei während der ersten Hälfte merklich gekühlt werden muß. Man hält anschließend die Temperatur 2 Stunden bei 60°, setzt erforderlichenfalls 25 ml 35 %iges Wasserstoffperoxid nach und hält weitere 2 Stunden auf Temperatur. Man kühlt nach beendeter Oxidation das erhaltene Öl-Wasser-Gemisch auf 0-5°, wobei gegebenenfalls nach Animpfen Kristallisation einsetzt. Das erhaltene Produkt wird abgesaugt, in 1000 ml Eiswasser mit einem Schnellrührer zerkleinert, abgesaugt und mit 500 ml Eiswasser gewaschen. Das Produkt entspricht der Formel

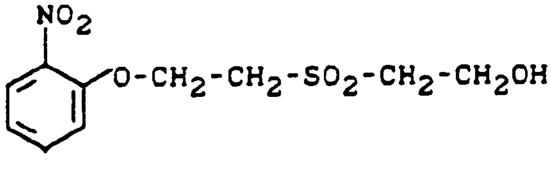

Fp.: 74-76° (Rohprodukt).

$^1$H-NMR in $D_6$-DMSO (TMS als innerem Standard).

δ = 3,36 ppm (2H, t)
δ = 3,69 ppm (2H, t)
δ = 3,80-3,88 ppm (2H, m)
δ = 4,54 ppm (2H, t)
δ = 5,14 ppm (1H, t)
δ = 7,14 ppm (1H, t?)
δ = 7,39-7,42 ppm (1H, d?)
δ = 7,62-7,68 ppm (1H, t?)
δ = 7,85-7,88 ppm (1H, d?)

80,0 g der erhaltenen o-Nitroverbindung werden in 600 ml Methanol in Gegenwart von 6 g Raney-Nickel unter einem Wasserstoffdruck von 10 bar in einer Stunde auf 60° erwärmt. Man hält einen Wasserstoffdruck von 10 bar laufend aufrecht, bis der Verbrauch zum Stillstand kommt. Nach Abkühlen und Entspannen wird die erhaltene Lösung vom Raney-Nickel abfiltriert und dann eingedampft. Man erhält ein kristallines Produkt der Formel

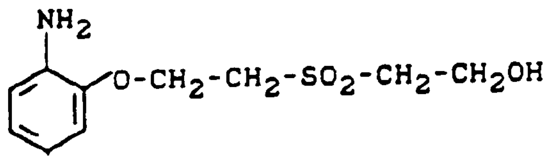

Eine aus 10 Volumina Isopropanol umkristalliserte Probe zeigt einen Schmelzpunkt von 79-81°.

$^1$H-NMR in $D_6$-DMSO (TMS als innerer Standard).

δ = 3,32 ppm (2H, t)
δ = 3,61 ppm (2H, t)
δ = 3,78-3,84 ppm (2H, q)
δ = 4,26 ppm (2H, t)
δ = 4,75 ppm (2H, s)
δ = 5,19 ppm (1H, t)
δ = 6,42-6,49 ppm (1H, m)
δ = 6,57-6,70 ppm (2H, m)
δ = 6,76-6,81 ppm (1H, dd)

## Beispiel 16

50 g o-Aminoverbindung der Formel

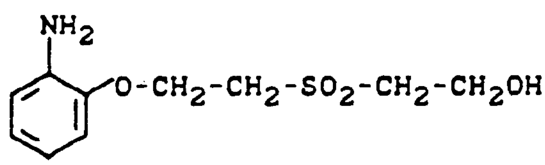

werden in 125 ml 96 %ige Schwefelsäure bei 0-5° eingetragen. Man rührt den Ansatz einige Stunden, bei 0-5°, bis das Edukt nur noch spurenweise vorhanden ist, eine chromatographische Probe keine zeitliche Veränderung im Reaktionsgemisch mehr anzeigt. Der Ansatz wird in 900 g Eis eingerührt, worauf das Reaktionsprodukt in Kristallen ausfällt. Man saugt den Niederschlag bei 0-5° ab und wäscht ihn mit Isopropanol oder 25 %iger Natriumchloridlösung sulfatfrei. Das erhaltene Produkt der Formel

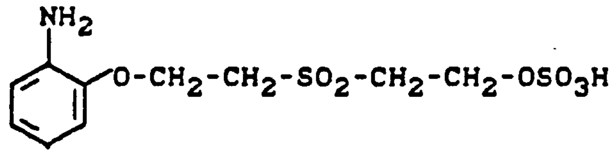

wird im Vakuum bei 50-60° getrocknet.

$^1$H-NMR in $D_6$-DMSO (TMS als innerer Standard).

δ = 3,54 ppm (2H, t)
δ = 3,67 ppm (2H, t)
δ = 4,15 ppm (2H, t)
δ = 4,44 ppm (2H, t)
δ = 6,94-7,04 ppm (1H, m)
δ = 7,22-7,37 ppm (3H, m)

Beispiel 17

40,0 g Aminoverbindung des Beispiels 3 der Formel

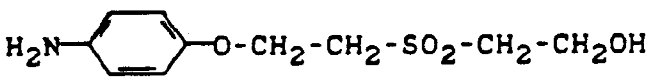

werden in 120 ml 20 %iges Oleum bei 15-20° eingetragen. Man rührt noch eine Stunde bei 20° nach, bis vollständige Kernsulfierung eingetreten ist und eine Probe kein Produkt des Beispiels 6 (nur Sulfatierung der Hydroxygruppe) mehr zeigt. Das Reaktionsgemisch wird in 200 g Eis gerührt und die Lösung mit 200 g Wasser verdünnt.

Man trägt in die Lösung bei unter 10° 220 g Calciumcarbonat ein, bis ein pH-Wert von 4,5 erreicht ist, saugt von ausgefallenem Calciumsulfat ab und wäscht den Filterkuchen mit etwa 500 ml Wasser frei von der Aminoverbindung. Das erhaltene Filtrat enthält die sehr leicht lösliche Verbindung der Formel

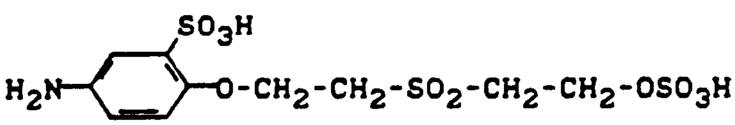

Das Filtrat kann, gegebenenfalls nach Eindampfen im Vakuum bis zum gewünschten Volumen, direkt umgesetzt werden, wie am Schluß des Beispiels 2 geschildert wurde.

Dampft man das Filtrat zur Trockene ein, so erhält man eine zähe, hygroskopische Masse, die erst nach scharfer Trocknung im Vakuum über Natriumhydroxid spröde und pulversierbar wird.

$^1$H-NMR in $D_2O$-$D_2SO_4$ (Trimethylsilylpropansulfonsäure, Na-Salz als innerer Standard, 250 MHz-Gerät)

δ = 3,83 ppm (2H, t)
δ = 3,90 ppm (2H, t)
δ = 4,45 ppm (2H, t)
δ = 4,62 ppm (2H, t)
δ = 7,23-7,26 (1H, d, $J_{ortho}$ 8 Hz)
δ = 7,52-7,55 ppm (1H, dd, $J_{ortho}$ 8 Hz, $J_{meta}$ 2 Hz)
δ = 7,78 ppm (1H, d, $J_{meta}$ 2 Hz)

Beispiel 18

70,0 g Aminoverbindung des Beispiels 15 der Formel

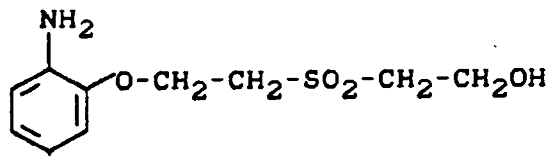

werden in 210 ml 20 %iges Oleum bei 20-25° unter leichter Außenkühlung eingetragen. Nach dreistündigem Rühren gibt man die Lösung in 1600 g Eis. Man verdünnt mit 1000 ml Wasser und trägt dann unterhalb von 10° etwa 395 g Calciumcarbonat ein, bis ein pH-Wert von 5,0 erreicht ist. Das Calciumsulfat wird abgesaugt, mit Wasser gewaschen, bis der Niederschlag frei von aromatischer Aminoverbindung ist. Nach Eindampfen der Filtrate im Vakuum fällt die Verbindung der Formel

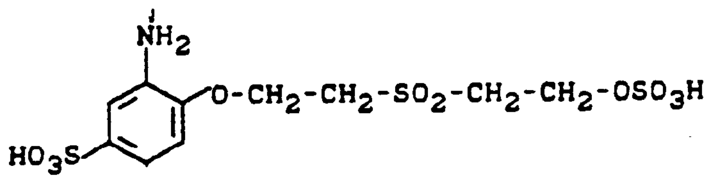

als leicht in Wasser lösliches Calciumsalz in Form eines gut trocknenden, weißen Rückstandes an.

$^1$H-NMR in $D_2O$-$D_2SO_4$ (Trimethylsilylpropansulfonsäure, Na-Salz als innerer Standard, 250 MHz-Gerät)

$\delta$ = 3,74 ppm (2H, t)
$\delta$ = 3,88 ppm (2H, t)
$\delta$ = 4,49 ppm (2H, t)
$\delta$ = 4,72 ppm (2H, t)
$\delta$ = 7,30-7,34 (1H, d, $J_{ortho} \approx$ 10 Hz)
$\delta$ = 7,79 ppm (1H, d, $J_{meta} \approx$ 2 Hz)
$\delta$ = 7,83-7,87 ppm (1H, dd, $J_{ortho} \approx$ 10 Hz, $J_{meta} \approx$ 2 Hz)

Beispiel 19

50,0 g des im Beispiel 13 erhaltenen Öles der Formel

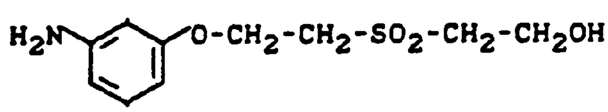

werden in 150 ml 30 %iges Oleum bei 15-20° eingetragen. Man rührt über Nacht. Wenn eine chromatographische Probe das Verschwinden des isomeren Kern-monosulfonsäure-Gemisches anzeigt und eine einheitliche Kern-disulfonsäure entstanden ist, gibt man den Ansatz in 1200 g Eis, verdünnt die erhaltene Lösung mit 1000 ml Wasser und trägt bei unter 10° etwa 270 g Calciumcarbonat ein, bis sich im Gemisch ein pH-wert von 5,0 einstellt.

Man trennt das Calciumsulfat durch Filtration ab, wäscht es mit Wasser frei von aromatischer Aminosulfonsäure und dampft die Filtrate im Vakuum ein. Nach Trocknung im Vakuum bei 40° über Natriumhydroxid erhält man das Calciumsalz der Verbindung

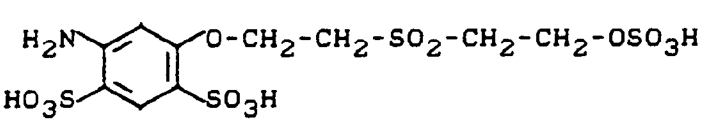

als stark hygroskopisches, an der Luft zerfließendes Pulver.

$^1$H-NMR in $D_2O$-$D_2SO_4$ (Trimethylsilylpropansulfonsäure, Na-Salz als innerer Standard).

$\delta$ = 3,84 ppm (2H, t nicht aufgelöst)
$\delta$ = 3,91 ppm (2H, t nicht aufgelöst)

δ = 4,47 ppm (2H, t nicht aufgelöst)
δ = 4,59 ppm (2H, t nicht aufgelöst)
δ = 6,77 ppm (1H, s)
δ = 8,08 ppm (1H, s)

Beispiel 20

40,0 g 1-Acetylamino-4-(2-chlorethoxy)-benzol werden mit 19,0 g 2-Mercaptoethanol und 12,8 g pulverisiertem Kaliumhydroxid in 150 ml Ethanol gekocht, bie eine chromatographische Probe den vollständigen Austausch des Chloratoms im Edukt gegen den β-Hydroxyethylmercaptid-Rest anzeigt.

Die erhaltene Suspension wird mit 300 ml Wasser verdünnt und die Mischung mit einer neutralisierten Lösung von 0,5 g Wolframsäure in 10 ml Wasser und 2,0 g Natriumacetat versetzt. Anschließend wird der pH-Wert mit Essigsäure auf 5,5 gestellt.

Zur Oxidation des Sulfids läßt man bei 50-60° 49 ml 35 %iges Wasserstoffperoxid im Laufe von einer Stunde zutropfen und hält dann die Temperatur noch etwa 3 Stunden auf 60°, bis die Oxidation des als Zwischenprodukt entstehenden Sulfoxides zum Sulfon vollständig ist.

Nach Abdestillation des Ethanols im Vakuum wird das entstandene Sulfon der Formel

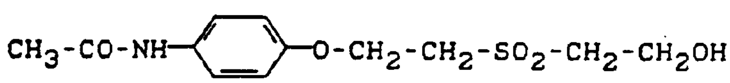

durch Filtration isoliert.
Fp. 107-108°C.
$^1$H-NMR in $D_6$-DMSO (TMS als innerer Standard).
δ = 2,02 ppm (3H,s)
δ = 3,32 ppm (2H,t)
δ = 3,62 ppm (2H,t)
δ = 3,84 ppm (2H,m)
δ = 4,32 ppm (2H,t)
δ = 5,16 ppm (1H,s)
δ = 6,92 ppm (2H,d)
δ = 7,50 ppm (2H,d)
δ = 9,81 ppm (1H,s)

Kochen des Produktes in 10 Volumina 10 %iger Salzsäure, Abkühlen der erhaltenen Lösung, Neutralisation mit Natronlauge und Absaugen des Niederschlages ergibt die gleiche Aminoverbindung wie im Beispiel 3 oder 4.

Beispiel 21

40,0 g des im Beispiel 13 erhaltenen Öles der Formel

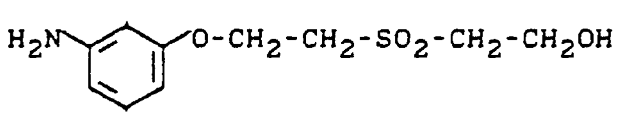

werden in eine Mischung von 50 ml 20 %igem Oleum und 50 ml 96 %iger Schwefelsäure bei 0-10° eingetragen. Man setzt anschließend noch 50 ml 20 %iges Oleum zu dem Reaktionsgemisch und hält dann die Temperatur etwa 5 Stunden auf 20-22°, bis eine chromatographische Probe vollständige Monosulfonierung im aromatischen Kern anzeigt. Der Ansatz wird in 1250 g Eis eingerührt und die erhaltene Lösung mit 1000 ml Wasser verdünnt.

Man neutralisiert die Schwefelsäure durch langsames Eintragen von etwa 270 g Calciumcarbonat bis zum pH-Wert 5,0, filtriert vom Calciumsulfat ab, wäscht mit Wasser, bis der Filterkuchen frei von diazotierbarem Material ist und dampft die vereinigten Filtrate im Vakuum zur Trockne ein. Man erhält die Calciumsalze eines Gemisches stellungsisomerer Sulfonsäuren der Formeln

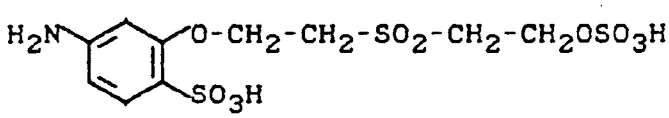

und

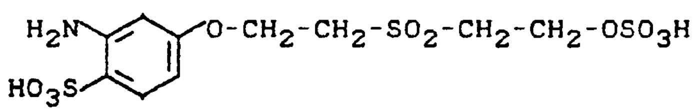

$^1$H-NMR des Isomeren-Gemisches in $D_2O$-$D_2SO_4$ (Trimethylsilylpropansulfonsäure, Na-Salz als innerer Standard)

δ = 3,76 - 3,93 ppm (4H, 4 Banden)
δ = 4,50 - 4,66 ppm (4H, 4 Banden)
δ = 7,14 - 7,23 ppm (2H, 3 Banden)
δ = 7,85 - 7,92 ppm (1H, 3 Banden)

Beispiel 22

275 g 4-Nitro-2-hydroxy-toluol werden in 2500 ml Wasser mit 260 ml 2n Natronlauge bei pH 8,5 und 80° gelöst. Man läßt 145 g 2-Chlorethanol zulaufen, hält im Reaktionsgemisch den pH-Wert weiter auf 8,5 und die Temperatur bei 80°. Unter den gleichen Bedingungen setzt man nach 3 Stunden noch 65 g 2-Chlorethanol und im Abstand von jeweils 2 Stunden noch viermal weiterhin je 65 g 2-Chlorethanol nach, bis das Edukt chromatographisch nicht mehr nachweisbar ist. Die erhaltene Emulsion liefert nach Erkalten unter Rühren grobe rechteckige Kristalle, die abgesaugt, mit 2 l Wasser gewaschen und im Vakuum getrocknet werden. Ausbeute 344 g.
Fp. 103-104°.

343 g des erhaltenen Produktes werden in 1000 ml Thionylchlorid in 30 Minuten eingetragen. Nach Abklingen der Gasentwicklung setzt man 10 ml Thionylchlorid zu und erhitzt in etwa 15 Minuten bis zum Rückfluß. Man kocht 2 Stunden am Rückfluß, kühlt nach beendeter Umsetzung auf Raumtemperatur ab und trägt die Lösung in 9,5 kg Eis ein. Man saugt das ausgefallene Produkt ab, wäscht es mit 7 l Wasser und nach Homogenisierung mit einem Schnellrührer nochmals mit Wasser bis zum neutralen Ablauf. Nach Trocknung im Vakuum bei Raumtemperatur erhält man 373 g eines Produktes der Formel

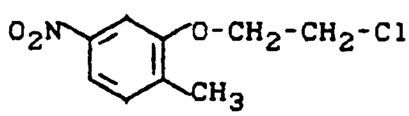

mit einem Schmelzpunkt von 65°.

373 g des erhaltenen 2-β-Chlorethoxy-4-nitrotoluols werden in 1600 ml Acetonitril unter Stickstoffatmosphäre mit 274 g Kaliumcarbonat und 149 g 2-Mercaptoethanol auf 70° erhitzt. Nach 7 Stunden setzt man 37 g Kaliumcarbonat und 37 g 2-Mercaptoethanol nach und wiederholt dieses nach weiteren 4 Stunden. Nach beendeter Umsetzung wird der Ansatz in 6,2 l Wasser gegossen. Aus der erhaltenen Emulsion kann nach Absetzen ein Öl abgetrennt und die wäßrige Phase mit Methylenchlorid extrahiert werden. Vereinigung von Extrakt und abgetrenntem Öl, waschen der erhaltenen Lösung mit Wasser, Trocknen über Natriumsulfat und Abdestillation des Methylenchlorids zuletzt im Vakuum bei 70° und 18 mbar ergibt 440 g eines Öles der Formel

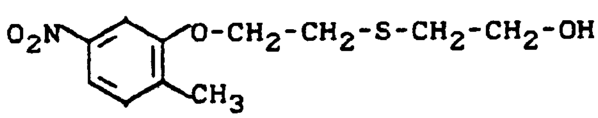

$^1$H-NMR in $D_6$-DMSO (TMS als innerer Standard)

δ = 2,28 ppm (3H,s)
δ = 2,72 ppm (2H,t)
δ = 2,98 ppm (2H,t)
δ = 3,53-3,63 ppm (2H,m)
δ = 4,28 ppm (2H,t)
δ = 4,81 ppm (1H,t)
δ = 7,42-7,47 ppm (1H,d)
δ = 7,71 ppm (1H,d; schwache Aufspaltung)
δ = 7,72-7,78 ppm (1H,dd)

Zur Oxydation des oben erhaltenen Thioethers setzt man entweder die nach Eingießen in Wasser oben erhaltene Rohemulsion unter Umgehung der beschriebenen Methylenchloridextraktion oder das nach Extraktion erhaltene Öl ein, man verfährt in beiden Fällen analog. Im letzteren Falle emulgiert man 440 g des Thioethers in 3,8 l Wasser, setzt 23 g Natriumacetat zu, stellt mit 7,6 ml Eiswasser auf pH 5,8 und gibt eine Katalysatorlösung aus 3,5 g Wolframsäure zu, die nach den Angaben des Beispiels 13 hergestellt wird. Man läßt nun bei 55-60° unter gelegentlicher Kühlung im Laufe einer Stunde 300 ml 35 %iges Wasserstoffperoxid zutropfen und hält danach die Temperatur auf 60°. Nach 5 Stunden werden nochmals 120 ml Wasserstoffperoxid nachgesetzt. Wenn die Oxydation nach weiteren etwa 4 Stunden bei 60° beendet ist, wird die erhaltene Emulsion kalt gerührt und gegebenenfalls angeimpft. Die erhaltene Kristallmasse wird abgesaugt, mit eiskaltem Wasser gewaschen und bei 50° im Vakuum getrocknet. Das Produkt der Formel (445 g, Fp. 76-78°, roh)

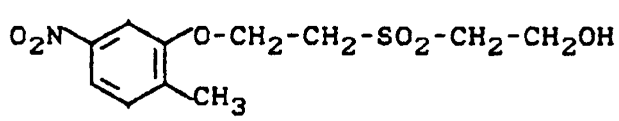

zeigt folgendes Kernresonanzspektrum:

$^1$H-NMR in $D_6$-DMSO (TMS als innerer Standard)
δ = 2,28 ppm (3H,s)
δ = 3,36 ppm (2H,t)
δ = 3,63 ppm (2H,t)
δ = 3,83-3,92 ppm (2H,m)
δ = 4,54 ppm (2H,t)
δ = 5,18 ppm (1H,t)
δ = 7,42-7,47 ppm (1H,d)
δ = 7,80-7,84 ppm (2H,m)

400 g der erhaltenen Nitroverbindung werden in 1800 ml Methanol nach Zusatz von 20 g Raney-Nickel bei einem Druck von 10 bar Wasserstoff bei 55-60° im Autoklaven hydriert. Die erhaltene Lösung wird bei 60° vom Raney-Nickel geklärt und das Filtrat im Rotationsverdampfer unter vermindertem Druck eingedampft. Man erhält als Rückstand das Amin der Formel

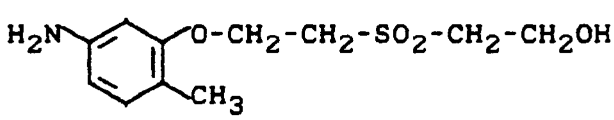

das einen Schmelzpunkt von 99-102° aufweist.

$^1$H-NMR in $D_6$-DMSO (TMS als innerer Standard)
δ = 1,98 ppm (3H,s)
δ = 3,32 ppm (2H,t)
δ = 3,60 ppm (2H,t)
δ = 3,78-3,84 ppm (2H,m)
δ = 4,19 ppm (2H,t)
δ = 4,84 ppm (2H, breit)
δ = 5,14 ppm (1H,t)
δ = 6,03-6,07 ppm (1H,dd)
δ = 6,19 ppm (1H,d)
δ = 6,69-6,73 ppm (1H,d)

Beispiel 23

Trägt man 100 g Aminoverbindung des Beispiels 22 in 250 ml 96 %ige Schwefelsäure bei 0° ein, rührt 17 Stunden bei dieser Temperatur, gibt die erhaltene Lösung in 400 g Eis, saugt die nach einigen Stunden ausgefallenen Kristalle ab und wäscht sie mit Isopropanol frei von Schwefelsäure, so erhält man den Schwefelsäurehalbester der Formel

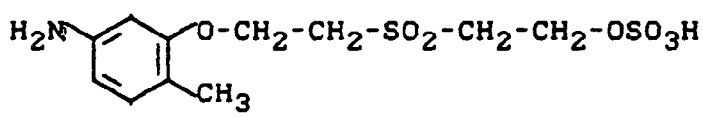

Er zeigt folgendes Kernresonanzspektrum:

$^1$H-NMR in $D_6$-DMSO (TMS als innerer Standard)

$\delta$ = 2,15 ppm (3H,s)
$\delta$ = 3,50 ppm (2H,t)
$\delta$ = 3,66 ppm (2H,t)
$\delta$ = 4,12 ppm (2H,t)
$\delta$ = 4,36 ppm (2H,t)
$\delta$ = 6,82-6,85 ppm (1H,dd)
$\delta$ = 7,93 ppm (1H,d)
$\delta$ = 7,21-7,24 ppm (1H,d)

Beispiel 24

100 g der Aminoverbindung des Beispiels 22 werden in 300 ml 20 %iges Oleum allmählich eingetragen. Man rührt 5 Stunden bei 20° nach, bis alles gelöst ist. Man gibt die erhaltene Lösung in 1200 g Eis, neutralisiert die Lösung mit ca. 580 g Calciumcarbonat bis zum pH 5,5, filtriert den ausgefallenen Gips ab und wäscht ihn mit Wasser frei von der Aminosulfonsäure. Die Filtrate werden im Vakuum eingedampft bis zur Trockene. Das erhaltene Produkt ist das Calciumsalz einer Sulfonsäure der Formel

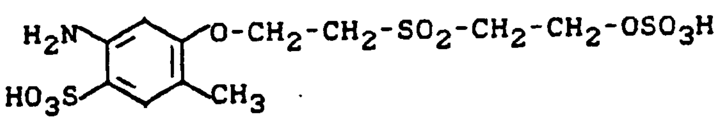

$^1$H-NMR in $D_2O$ + $D_2SO_4$ (Trimethylsilylpropansulfonsäure, Na-Salz als innerer Standard)

$\delta$ = 2,18 ppm (3H,s)
$\delta$ = 3,73 ppm (2H,t unscharf)
$\delta$ = 3,82 ppm (2H,t unscharf)
$\delta$ = 4,47-4,53 ppm (4H, mehrere Banden zusammenfallend)
$\delta$ = 6,97 ppm (1H,s)
$\delta$ = 7,59 ppm (1H,s)

**Patentansprüche**

1. Triphendioxazin-Reaktivfarbstoffe der allgemeinen Formel

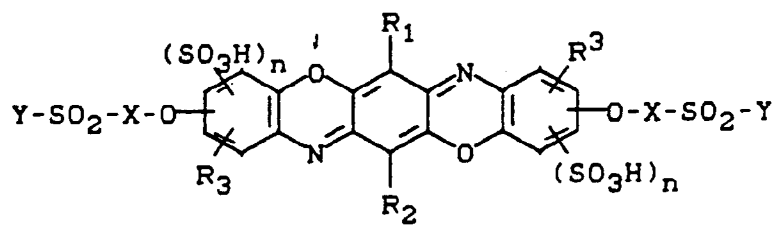

(I),

worin

X aliphatisches Brückenglied

Y $-CH=CH_2$, $-CH_2-CH_2-Z$,

worin

Z abspaltbare Gruppe wie $OSO_3H$, $S_2O_3H$, Cl, Br, $O-COCH_3$, $OPO_3H_2$, $\overset{\oplus}{N}(R_4)_3$ mit $R_4$ = $C_1$-$C_4$-Alkyl,

bevorzugt jedoch $OSO_3H$ ist,

n 0 - 2 vorzugsweise 1,

$R_1$, $R_2$ H, Halogen, $C_1$-$C_4$-Alkoxy, gegebenenfalls substituiertes Phenoxy oder Aryl, Acylamino, Carboxy, Carbalkoxy, vorzugsweise Cl,

$R_3$ H, $C_1$-$C_4$-Alkyl, $C_1$-$C_4$-Alkoxy, Halogen, Carboxy

bedeuten.

2. Farbstoffe des Anspruchs 1 der Formel

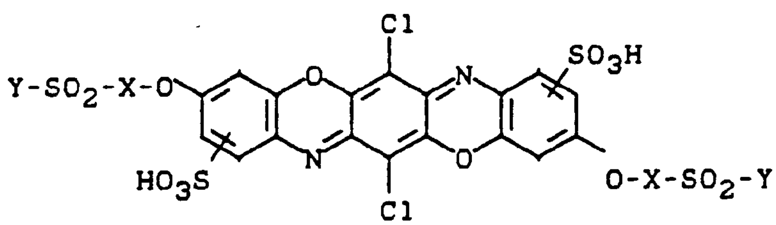

worin X und Y die in Anspruch 1 angegebene Bedeutung haben.

3. Farbstoffe des Anspruchs 1 der Formel

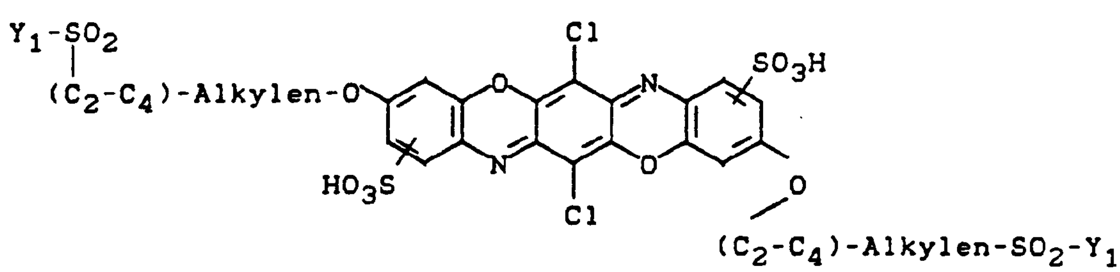

worin

$Y_1$ $CH_2-CH_2-OSO_3H$ oder $-CH=CH_2$

bedeutet.

4. Farbstoffe der Ansprüche 1-3 in denen die Sulfogruppen des Triphendioxazinrings in o-Stellung zum Rest $-O-X-SO_2-Y$ bzw. $-O-C_2-C_4$-Alkylen-$SO_2-Y_1$ stehen.

5. Verwendung der Farbstoffe der Ansprüche 1-4 zum Färben und Bedrucken von hydroxylgruppen- oder amidgruppenhaltigen Materialien.

6. Mit den Farbstoffen der Ansprüche 1-4 gefärbte oder bedruckte hydroxylgruppen- oder amidgruppenhaltigen Materialien.

7. Amine der Formeln

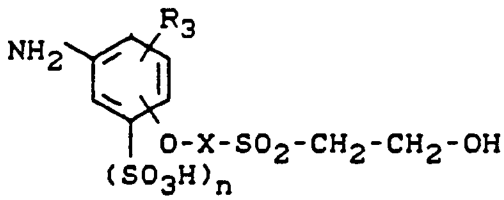

und

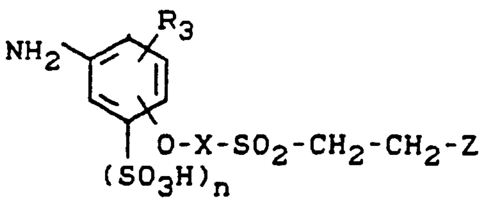

worin
Z, X, $R_3$ und n die in Anspruch 1 angegebene Bedeutung haben.

**Claims**

1. Triphendioxazine reactive dyestuffs of the general formula

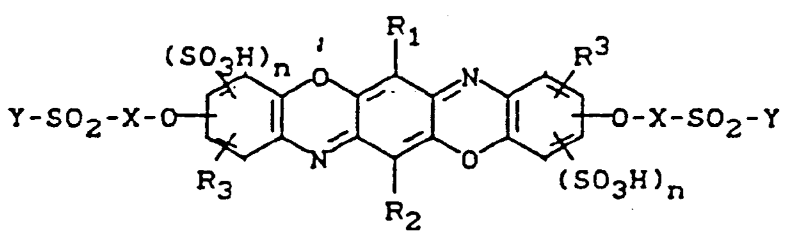

(I),

wherein
X denotes an aliphatic bridge member,
Y denotes $-CH=CH_2$ or $-CH_2-CH_2-Z$,
wherein
Z denotes a group which can be split off, such as
$OSO_3H$, $S_2O_3H$, Cl, Br, $O-COCH_3$, $OPO_3H_2$ or $\overset{\oplus}{N}(R_4)_3$, where $R_4$ = $C_1$-$C_4$-alkyl, but is preferably $OSO_3H$,
n denotes 0 - 2, preferably 1,
$R_1$ and $R_2$ denote H, halogen, $C_1$-$C_4$-alkoxy, optionally substituted phenoxy or aryl, acylamino, carboxyl or carbalkoxy, preferably Cl, and
$R_3$ denotes H, $C_1$-$C_4$-alkyl, $C_1$-$C_4$-alkoxy, halogen or carboxyl.

2. Dyestuffs of Claim 1 of the formula

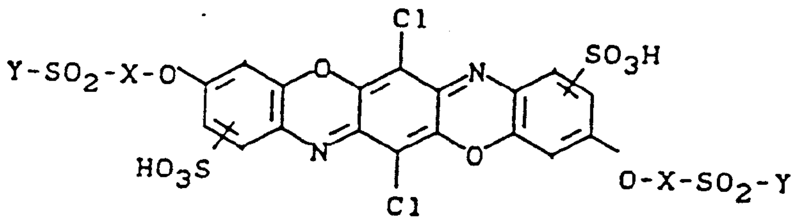

wherein X and Y have the meaning given in Claim 1.

**3.** Dyestuffs of Claim 1 of the formula

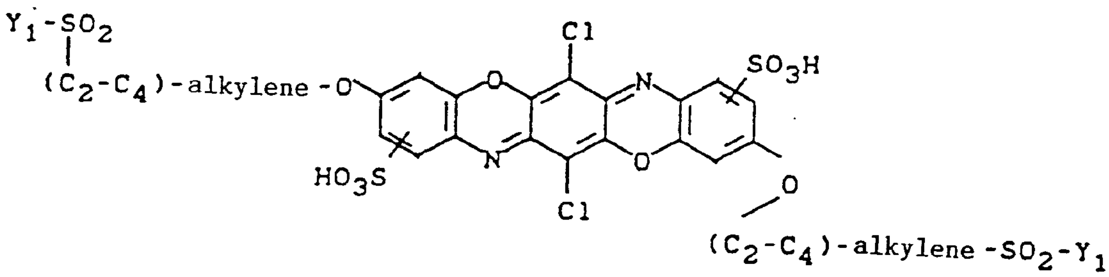

wherein
$Y_1$ denotes $CH_2$-$CH_2$-$OSO_3H$ or -CH=$CH_2$.

**4.** Dyestuffs of Claims 1-3 in which the sulpho groups of the triphendioxazine ring are in the o-position relative to the radical -O-X-$SO_2$-Y or -O-$C_2$-$C_4$-alkylene-$SO_2$-$Y_1$.

**5.** Use of the dyestuffs of Claims 1-4 for dyeing and printing materials containing hydroxyl groups or amide groups.

**6.** Materials containing hydroxyl groups or amide groups which have been dyed or printed with the dyestuffs of Claims 1-4.

**7.** Amines of the formulae

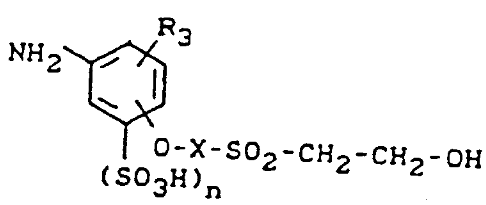

and

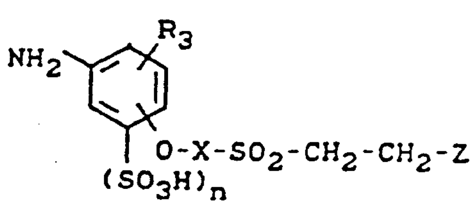

wherein
Z, X, $R_3$ and n have the meaning given in Claim 1.

## Revendications

1. Colorants réactifs de triphénodioxazines, de formule générale

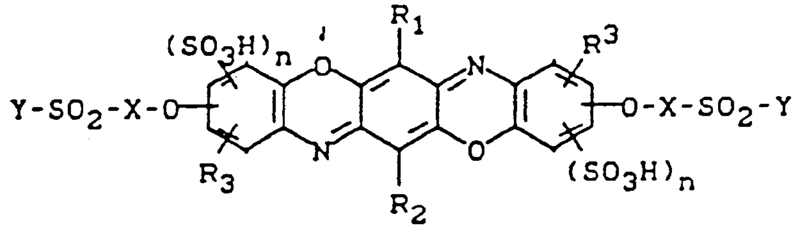

(I),

dans laquelle

X représente un pont aliphatique,

Y représente $-CH=CH_2$, $-CH_2-CH_2-Z$ dans lequel

Z représente un substituant éliminable tel que $OSO_3H$, $S_2O_3H$, Cl, Br, $O-COCH_3$, $OPO_3H_2$, $\overset{+}{N}(R_4)_3$ avec $R_4$ = alkyle en $C_1$-$C_4$,

de préférence cependant $OSO_3H$,

n a une valeur de 0 à 2 et est de préférence égal à 1,

$R_1$, $R_2$ représentent chacun H, un halogène, un groupe alcoxy en $C_1$-$C_4$, un groupe phénoxy éventuellement substitué ou un groupe aryle, acylamino, carboxy, carbalcoxy, de préférence Cl,

$R_3$ représente H, un groupe alkyle en $C_1$-$C_4$, alcoxy en $C_1$-$C_4$, un halogène ou un groupe carboxy.

2. Colorants de la revendication 1, de formule

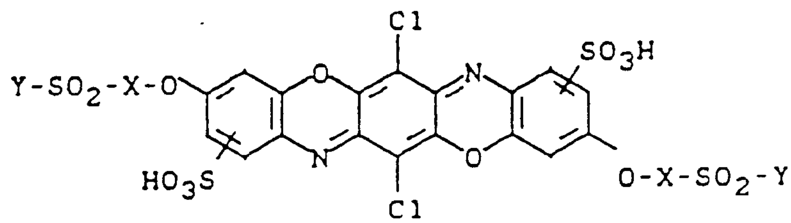

dans laquelle X et Y ont les significations indiquées dans la revendication 1.

3. Colorants de la revendication 1, de formule

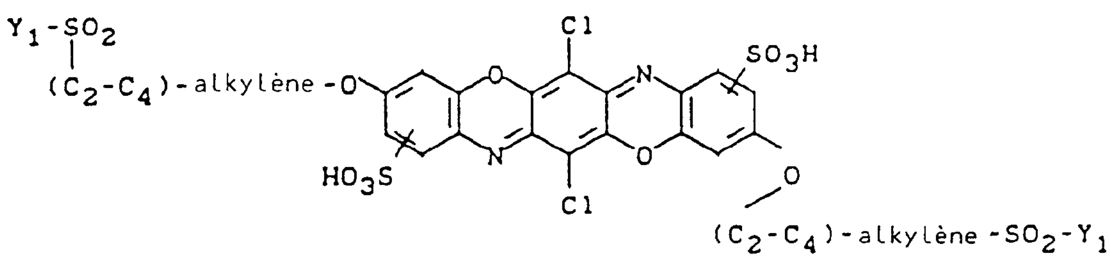

dans laquelle

$Y_1$ représente $CH_2$-$CH_2$-$OSO_3H$ ou $-CH=CH_2$.

4. Colorants des revendications 1 à 3, dans lesquels les groupes sulfo du cycle triphénodioxazine sont en position ortho du groupe $-O-X-SO_2-Y$ et du groupe -O-alkylène en $C_2$-$C_4$ $-SO_2-Y_1$ respectivement.

5. Utilisation des colorants des revendications 1 à 4 pour la teinture et l'impression de matières contenant des groupes hydroxy ou des groupes amide.

6. Matières contenant des groupes hydroxy ou des groupes amide, teintes ou imprimées à l'aide des colorants des revendications 1 à 4.

7. Amines de formule

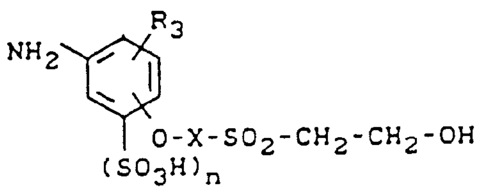

et

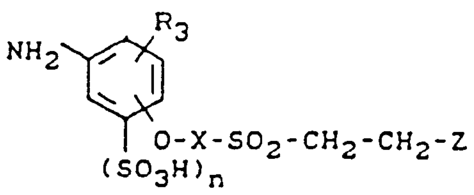

dans lesquelles Z, X, $R_3$ et n ont les significations indiquées dans la revendication 1.